(19) Europäisches Patentamt
European Patent Office
Office européen des brevets

(11) Publication number: **0 517 262 A1**

(12) **EUROPEAN PATENT APPLICATION**

(21) Application number: **92109574.1**

(22) Date of filing: **05.06.92**

(51) Int. Cl.[5]: **C07H 19/06**, C07H 19/10, A61K 31/70

(30) Priority: **07.06.91 JP 136046/91**

(43) Date of publication of application: **09.12.92 Bulletin 92/50**

(84) Designated Contracting States: **DE FR GB IT**

(71) Applicant: **MITSUI TOATSU CHEMICALS, Inc.**
**2-5 Kasumigaseki 3-chome**
**Chiyoda-Ku Tokyo 100(JP)**

(72) Inventor: **Fukazawa, Nobuyuki**
**2-25-17 Tobudai**
**Mobara-shi, Chiba-ken 297(JP)**
Inventor: **Fujiwara, Junya**
**16 Miyanodai-Daini, 2142, Tougou**
**Mobara-shi, Chiba-ken 297(JP)**
Inventor: **Komatsu, Hironori**
**125 Tonodai, 90-1 Machibo**
**Mobara-shi, Chiba-ken 297(JP)**
Inventor: **Kawauchi, Nobuya**
**74 Myanodai-Ryo, 2142 Tougou**
**Mobara-shi, Chiba-ken 297(JP)**
Inventor: **Yano, Osamu**
**740-11 Miyanodai-Daini Apt., 2142 Togo**
**Mobara-shi, Chiba-ken 297(JP)**
Inventor: **Iwata, Daiji**
**212 Mutsuno, 2791-1 Mutsuno**
**Mobara-shi, Chiba-ken 297(JP)**
Inventor: **Nakanishi, Osamu**
**640-21 Miyanodai-Apt., 2141 Togo**
**Mobara-shi, Chiba-ken 297(JP)**
Inventor: **Edatsugi, Hajime**
**454 Miyanodai-Ryo, 2142 Tougou**
**Mobara-shi, Chiba-ken 297(JP)**

(74) Representative: **Vossius & Partner**
**Siebertstrasse 4 P.O. Box 86 07 67**
**W-8000 München 86(DE)**

(54) **Trifluorothymidine derivatives, process for producing the same and anti-cancer agent containing the same.**

(57) Novel trifluorothymidine derivatives having anti-cancer activities are disclosed. The trifluorothymidine derivatives of the present invention are represented by the formula [I]:

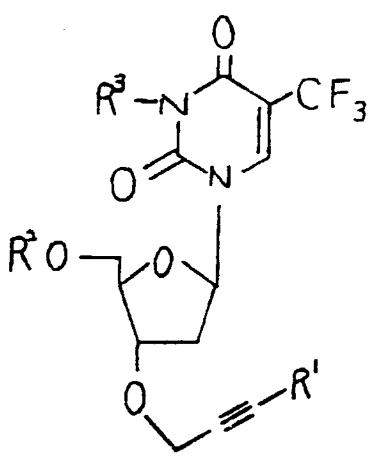

[I]

EP 0 517 262 A1

(wherein $R^1$ represents hydrogen atom or $C_1$ - $C_4$ alkyl group; $R^2$ represents hydrogen atom, $C_1$ - $C_{30}$ saturated or unsaturated alkyl-substituted carbonyl group, alkyl-substituted benzoyl group, $C_1$ - $C_4$ alkyloxycarbonyl group; dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group; $R^3$ represents hydrogen atom, $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group).

This invention relates to novel trifluorothymidine derivatives having anti-cancer activities.

Trifluorothymidine (hereinafter also referred to as "F3Thd") of the following formula is a nucleic acid metabolism antagonist first synthesized by Heidelberger et al (Journal of the American Chemical Society,Vol. 84, p.3597, 1962).

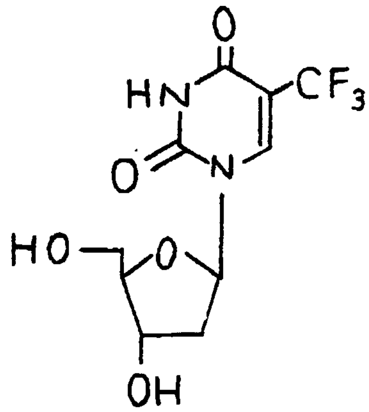

The pharmacological properties of this compound has been studied. It is known that this compound has anti-viral activity rather than anti-cancer activity and is effective against infectious diseases caused by, for example, herpes virus and vaccinia virus. As for anti-tumor activity, it has been reported that this compound exhibits excellent anti-tumor activity against experimental model tumors such as L1210 leukemia and adenocarcinoma-755 (Cancer Research, Vol. 24, p.1979, 1964). However, since this compound is not well absorbed when administered orally and is rapidly metabolized in the body, in the clinical applications, the anti-tumor activity of this compound is not so high as that shown in in vitro studies using the model tumors. Further, this compound has side effects such as inhibition of bone marrow.

Thus, a novel F3Thd derivative is desired, which has higher anti-tumor activity in vivo and which has less side effects than F3Thd.

F3Thd derivatives having anti-tumor activities are disclosed in JP-A-58-152898, -59-36696, -60-56996 and -62-187482 and Chemical Pharmaceutical Bulletin, Vol. 37, p.2287, 1989. However, the above-mentioned desire is not well satisfied with these F3Thd derivatives. Particularly, these known F3Thd derivatives are rapidly metabolized (conjugation and the like) and excreted, so that the pharmacological effect given by the active moiety F3Thd is not sustained for a long time. Thus, no F3Thd derivatives which are useful in clinical applications are known.

Accordingly, an object of the present invention is to provide a novel F3Thd derivative which is more absorbed when administered orally and less metabolized (conjugation and the like) and less excreted than F3Thd and the known derivatives thereof, so that the kinetics of the drug and its active moiety in the blood are improved and so the anti-tumor activity in vivo is promoted, and which has less side effects than the conventional F3Thd and derivatives thereof.

Another object of the present invention is to provide an anti-cancer agent containing the novel F3Thd derivative of the present invention as an effective ingredient.

Still another object of the present invention is to provide processes for producing the novel F3Thd derivatives according to the present invention.

In order to develop a compound which is more absorbed when orally administered and less metabolized and less excreted and which has less side effects than F3Thd and known derivatives thereof, the present inventors tried to improve the kinetics of the compound in the blood while keeping the anti-tumor activity of F3Thd per se. More particularly, the present inventors tried to develop a compound which reversibly releases F3Thd in vivo, that is, a prodrug of F3Thd. For this, the present inventors introduced various substituents on 3'- and 5'-positions of the deoxyribose in F3Thd. As a result, as will be described later in detail, it was surprisingly discovered that a group of novel F3Thd derivatives in which 3'-position is substituted with propargyl group show much higher absorption when administered orally and much higher anti-tumor activities than the known derivatives. This is presumably because that the propargyl group is slowly liberated by an enzyme and so the active moiety F3Thd is slowly released far a long time, so that the rates of metabolism such as conjugation and excretion are slowed accordingly. Further, since the F3Thd derivatives of the present invention slowly release F3Thd, the side effects such as damages to alimentary canal are largely reduced. The present invention is based on this discovery.

That is, the present invention provides a trifluorothymidine derivative of the formula [I]:

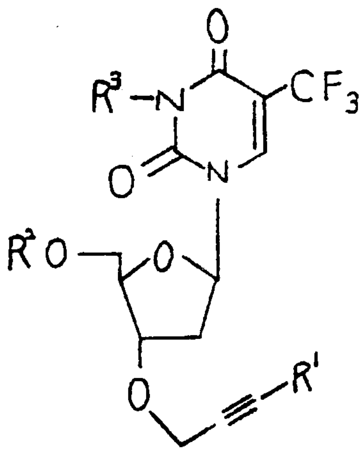

[I]

(wherein $R^1$ represents hydrogen atom or $C_1$ - $C_4$ alkyl group; $R^2$ represents hydrogen atom, $C_1$ - $C_{30}$ saturated or unsaturated alkyl-substituted carbonyl group, $C_1$ - $C_{10}$ alkyl-substituted benzoyl group, $C_1$ - $C_4$ alkyloxycarbonyl group; dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group; $R^3$ represents hydrogen atom, $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group).

The present invention also provides an anti-cancer agent comprising effective amount of the trifluorothymidine derivative of the present invention and a pharmaceutically acceptable carrier.

The present invention further provides a process for producing a trifluorothymidine derivative of the formula [Ia]:

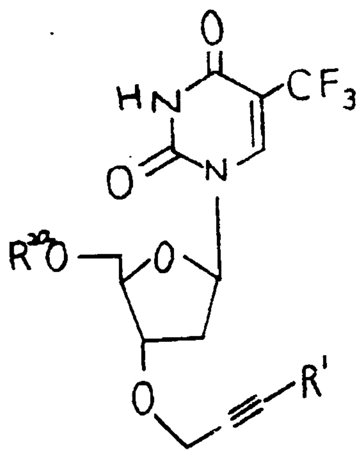

[Ia]

(wherein $R^1$ represents the same meaning as in formula [I], $R^{2a}$ represents the same meaning as in formula [I] except that it is not hydrogen atom)
comprising reacting a trifluorothymidine derivative of the formula [III]:

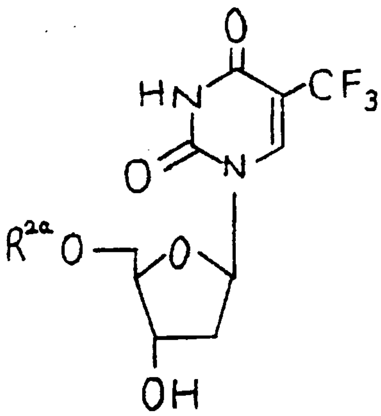

[III]

(wherein $R^{2a}$ represents the same meaning as in formula [Ia]) with a compound of the formula [IV]:

$R^1C{\equiv}C\text{-}CH_2\text{-}X$ [IV]

(wherein $R^1$ represents the same meaning as in formula [I] and X represents halogen) in the presence of a base.

The present invention still further provides a process for producing a trifluorothymidine derivative of the formula [Ib]:

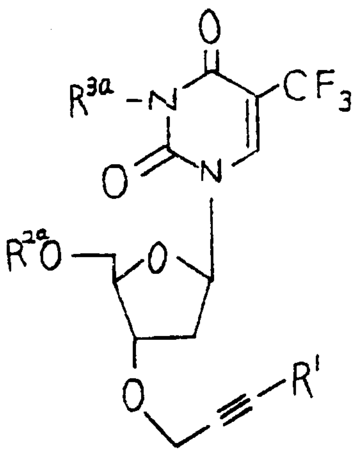

[Ib]

(wherein $R^1$ represents the same meaning as in formula [I], $R^{2a}$ represents the same meaning as in formula [Ia], $R^{3a}$ represents $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group) comprising substituting for the hydrogen atom attached to the nitrogen atom at 3-position of the trifluorothymidine derivative of the formula [Ia] a $C_1$ - $C_4$ alkyl group, benzyl group, benzoyl group, $C_1$ - $C_4$ alkyloxy-substitutedbenzoyl group, furoyl group, $C_1$ - $C_4$ alkyloxymethyl group or $C_1$ - $C_4$ alkyl-substituted carbonyl group.

The present invention still further provides a process for producing a trifluorothymidine derivative of the formula [Ic]:

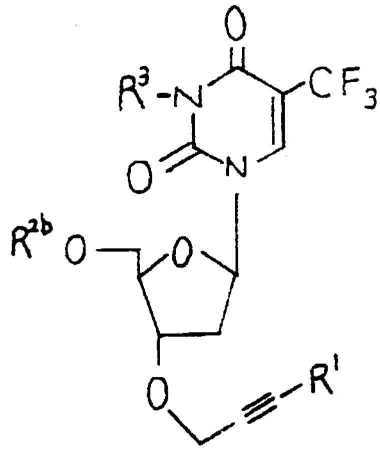

[Ic]

(wherein $R^1$ and $R^3$ represent the same meanings as in formula [I], $R^{2b}$ represents hydrogen atom) comprising reacting a trifluorothymidine derivative of the formula [Id]:

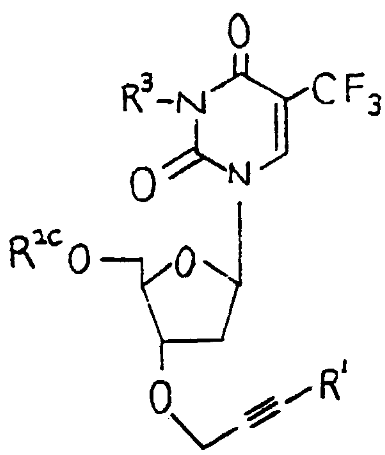

[Id]

(wherein $R^1$ and $R^3$ represent the same meanings as in formula [I], $R^{2c}$ represents $C_1$ - $C_{20}$ alkyl-substituted silyl group, silyl group substituted with $C_1$ - $C_{10}$ alkyl group and/or phenyl group, or trityl group) with an acid or fluoride ion so as to convert $R^{2c}$ in said trifluorothymidine derivative represented by the formula [Id] to hydrogen atom.

The present invention still further provides a process for producing a trifluorothymidine derivative of the formula [Ie]:

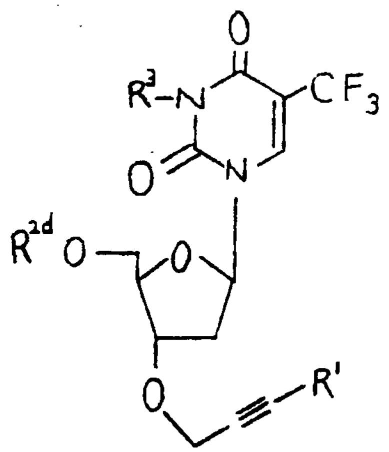

[Ie]

(wherein $R^1$ and $R^3$ represent the same meanings as in formula [I], $R^{2d}$ represents the same meaning as $R^2$ in formula [I] except that it is not hydrogen atom)

comprising substituting for the hydrogen atom represented by $R^{2b}$ of the trifluorothymidine derivative of the formula [Ic] a $C_1$ - $C_{30}$ saturated or unsaturated alkyl-substituted carbonyl group, alkyl-substituted benzoyl group, $C_1$ - $C_4$ alkyloxycarbonyl group, dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group.

Since the F3Thd derivatives of the present invention are slowly metabolized while slowly releasing F3Thd in vivo, the anti-cancer activity of F3Thd can be exhibited for a long time, and side effects by F3Thd are small. Thus, by the present invention, F3Thd derivatives which are useful for inhibiting cancers in clinical applications were first provided.

Fig. 1 shows the change in the concentration of the compound of the present invention in blood with time; and

Fig. 2 shows the increase in life span of the mice administered with the compound of the present invention or F3Thd.

The trifluorothymidine derivatives of the present invention will now be described in detail.

As mentioned above, the trifluorothymidine derivatives of the present invention are represented by the above-described formula [I]. In formula [I], $R^1$ represents hydrogen atom or $C_1$ - $C_4$ alkyl group. Examples of the $C_1$ - $C_4$ alkyl group include methyl, ethyl, n-propyl, isopropyl, n-butyl, isobutyl and t-butyl groups.

In formula [I], $R^2$ represents hydrogen atom, $C_1$ -$C_{30}$ saturated or unsaturated alkyl-substituted carbonyl group, $C_1$ - $C_{10}$ alkyl-substituted benzoyl group, $C_1$ - $C_4$ alkyloxycarbonyl group, dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group. Preferred examples of $C_1$ - $C_{30}$ saturated or unsaturated alkyl-substituted carbonyl group include $C_1$ - $C_4$ lower alkyl-substituted carbonyl groups such as acetyl, propionyl, pivaloyl, butyryl, valeryl; hexanoyl, octanoyl, nonanoyl, decanoyl, lauroyl, myristoyl, palmitoyl, stearoyl, eicosanoyl, docosanoyl, tetracosanoyl, hexacosanoyl, octacosanoyl, crotonoyl, 9-hexadecenoyl, oleoyl, 11-octadecenoyl, 9,12-octadecadienoyl, 6,9,12-octadecatrienoyl, 9,11,13-octadecatrienoyl, 8,11-eicosadienoyl, 5,8,11-eicosatrienoyl, 5,8,11,14-eicosatetraenoyl and 15-tetracosaenoyl groups. Preferred examples of $C_1$ - $C_{10}$ alkyl-substituted benzoyl group include 4-methylbenzoyl, 4-ethylbenzoyl, 4-propylbenzoyl, 4-butylbenzoyl, 4-pentylbenzoyl, 4-hexylbenzoyl, 4-heptylbenzoyl, 4-octylbenzoyl, 4-nonylbenzoyl and 4-decylbenzoyl groups. Preferred examples of $C_1$ - $C_4$ alkyloxycarbonyl group include methoxycarbonyl, ethoxycarbonyl, propoxycarbonyl and butoxycarbonyl groups. Preferred examples of $C_1$ - $C_4$ alkyloxymethyl group include methoxymethyl group and ethoxymethyl group. Preferred examples of $C_1$ - $C_{20}$ alkyl-substituted silyl group include $C_1$ - $C_4$ lower alkyl-substituted silyl groups such as trimethylsilyl, ethyldimethylsilyl, triethylsilyl, propyldimethylsilyl, isopropyldimethylsilyl, tripropylsilyl, triisopropylsilyl, butyldimethylsilyl, dibutylmethylsilyl, tributylsilyl, triisobutylsilyl, isobutyldimethylsilyl, tert-butyldimethylsilyl, di-tert-butylmethylsilyl; pentyldimethylsilyl, (2,3-dimethylpropyl)dimethylsilyl, hexyldimethylsilyl, cyclohexyldimethylsilyl, trihexylsilyl, (3,3-dimethylbutyl)-dimethylsilyl, (1,1,2-trimethylpropyl)dimethylsilyl, heptyldimethylsilyl, octyldimethylsilyl, nonyldimethylsilyl, decyldimethylsilyl, dodecyldimethylsilyl, tetradecyldimethylsilyl, hexadecyldimethylsilyl, octadecyldimethylsilyl and eicosyldimethylsilyl groups. Preferred examples of silyl group substituted with $C_1$ -$C_{10}$ alkyl group and/or phenyl group include diphenylmethylsilyl, phenyldimethylsilyl, triphenylsilyl, tert-butyldiphenylsilyl and diphenylbutylsilyl groups. Preferred examples of $C_1$ - $C_{30}$ cyclic or chain alkyl-substituted carbamoyl group include N-cyclopentylcarbamoyl, N-cyclohexylcarbamoyl, N-cycloheptylcarbamoyl, N-cyclooctylcarbamoyl, N-cyclopentyl-N-methylcarbamoyl, N-cyclohexyl-N-methylcarbamoyl, N-cycloheptyl-N-methylcarbamoyl, N-cyclooctyl-N-methylcarbamoyl, N-cyclopentyl-N-ethylcarbamoyl, N-cyclohexyl-N-ethylcarbamoyl, N-cycloheptyl-N-ethylcarbamoyl, N-cyclooctyl-N-ethylcarbamoyl, N-cyclopentyl-N-propylcarbamoyl, N-cyclohexyl-N-propylcarbamoyl, N-cycloheptyl-N-propylcarbamoyl, N-cyclooctyl-N-propylcarbamoyl, N-cyclopentyl-N-butylcarbamoyl, N-cyclohexyl-N-butylcarbamoyl, N-cycloheptyl-N-butylcarbamoyl, N-cyclooctyl-N-butylcarbamoyl, N-methylcarbamoyl, N-ethylcarbamoyl, N-propylcarbamoyl, N-butylcarbamoyl, N-pentylcarbamoyl, N-hexylcarbamoyl, N-heptylcarbamoyl, N-octylcarbamoyl, N-nonylcarbamoyl, N-dodecylcarbamoyl, N-tetradecylcarbamoyl, N-hexadecylcarbamoyl, N-octadecylcarbamoyl, N-eicosanylcarbamoyl, N-docosanylcarbamoyl, N-tetracosanylcarbamoyl, N-hexacosanylcarbamoyl, N-octacosanylcarbamoyl, N-triacontanylcarbamoyl, N,N-dimethylcarbamoyl, N-ethyl-N-methylcarbamoyl, N-propyl-N-methylcarbamoyl, N-butyl-N-methylcarbamoyl, N-pentyl-N-methylcarbamoyl, N-hexyl-N-methylcarbamoyl, N-heptyl-N-methylcarbamoyl, N-octyl-N-methylcarbamoyl, N-nonyl-N-methylcarbamoyl, N-dodecyl-N-methylcarbamoyl, N-tetradecyl-N-methylcarbamoyl, N-hexadecyl-N-methylcarbamoyl, N-octadecyl-N-methylcarbamoyl, N,N-diethylcarbamoyl, N-propyl-N-ethylcarbamoyl, N-butyl-N-ethylcarbamoyl, N-pentyl-N-ethylcarbamoyl, N-hexyl-N-ethylcarbamoyl, N-heptyl-N-ethylcarbamoyl, N-octyl-N-ethylcarbamoyl, N-nonyl-N-ethylcarbamoyl, N-dodecyl-N-ethylcarbamoyl, N-tetradecyl-N-ethylcarbamoyl, N-hexadecyl-N-ethylcarbamoyl, N-octadecyl-N-ethylcarbamoyl, N,N-dipropylcarbamoyl, N-butyl-N-propylcarbamoyl, N-pentyl-N-propylcarbamoyl, N-hexyl-N-propylcarbamoyl, N-heptyl-N-propylcarbamoyl, N-octyl-N-propylcarbamoyl, N-nonyl-N-propylcarbamoyl, N-dodecyl-N-propylcarbamoyl, N-tetradecyl-N-propylcarbamoyl, N-hexadecyl-N-propylcarbamoyl, N-octadecyl-N-propylcarbamoyl, N,N-dibutylcarbamoyl, N-pentyl-N-butylcarbamoyl, N-hexyl-N-butylcarbamoyl, N-heptyl-N-butylcarbamoyl, N-octyl-N-butylcarbamoyl, N-nonyl-N-butylcarbamoyl, N-dodecyl-N-butylcarbamoyl, N-tetradecyl-N-butylcarbamoyl, N-hexadecyl-N-butylcarbamoyl, N-octadecyl-N-butylcarbamoyl and N-{(3-(diethylamino)propyl}carbamoyl groups. Preferred examples of N-alkylpiperazinylacetyl group include N-methylpiperazinylacetyl, N-ethylpiperazinylacetyl, N-propylpiperazinylacetyl, N-butylpiperazinylacetyl, N-pentylpiperazinylacetyl, N-hexylpiperazinylacetyl, N-heptylpiperazinylacetyl, N-octylpiperazinylacetyl, N-nonylpiperazinylacetyl, N-decylpiperazinylacetyl, N-dodecylpiperazinylacetyl, N-tetradecylpiperazinylacetyl, N-hexadecylpiperazinylacetyl and N-octadecylpiperazinylacetyl. Preferred examples of N-alkylprolyl group include N-methyl-L-prolyl, N-ethyl-L-prolyl, N-propyl-L-prolyl, N-butyl-L-prolyl, N-pentyl-L-prolyl, N-hexyl-L-prolyl, N-heptyl-L-prolyl, N-octyl-L-prolyl, N-nonyl-L-prolyl, N-decyl-L-prolyl, N-dodecyl-L-prolyl, N-tetradecyl-L-prolyl, N-hexadecyl-L-prolyl and N-octadecyl-L-prolyl. Preferred examples of alkyl-substituted phosphoryl group include ethoxyhydroxyphosphoryl, butoxyhydroxyphosphoryl, hexyloxyhydroxyphosphoryl, octyloxyhydroxyphosphoryl, decyloxyhydroxyphosphoryl, dodecyloxyhydroxyphosphoryl, dibutoxyphosphoryl and 2-oxo-1,3-dioxaphospholan-2-yl groups.

In formula [I], $R^3$ represents hydrogen atom, $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group. Preferred examples of $C_1$ - $C_4$ alkyl group are methyl, ethyl, propyl and butyl groups. Preferred examples of $C_1$ - $C_4$ alkyloxy-substituted benzoyl group include 4-methoxybenzoyl and 4-ethoxybenzoyl groups. A preferred example of furoyl group is 2-furoyl group. Preferred examples of $C_1$ - $C_4$ alkyl-substituted carbonyl group include acetyl, propionyl, pivaloyl and butyryl groups.

Specific but non-limiting examples of the trifluorothymidine derivatives of the present invention include the following compounds:

3'-O-propargyl-α,α,α-trifluorothymidine,
5'-O-trimethylsilyl-3'-O-propargyl-α,α,α-trifluorothymidine,
5'-O-ethyldimethylsilyl-3'-O-propargyl-α,α,α-trifluorothymidine,
5'-O-triethylsilyl-3'-O-propargyl-α,α,α-trifluorothymidine,
5'-O-propyldimethylsilyl-3'-O-propargyl-α,α,α-trifluorothymidine,
5'-O-isopropyldimethylsilyl-3'-O-propargyl-α,α,α-trifluorothymidine,
5'-O-tripropylsilyl-3'-O-propargyl-α,α,α-trifluorothymidine,
5'-O-triisopropylsilyl-3'-O-propargyl-α,α,α-trifluorothymidine,
5'-O-butyldimethylsilyl-3,-O-propargyl-α,α,α-trifluorothymidine,

5'-O-dibutylmethylsilyl-3'-O-propargyl-α,α,α-trifluorothymidine,
5'-O-tributylsilyl-3'-O-propargyl-α,α,α-trifluorothymidine,
5'-O-triisobutylsilyl-3'-O-propargyl-α,α,α-trifluorothymidine,
5'-O-isobutyldimethylsilyl-3'-O-propargyl-α,α,α-trifluorothymidine,
5'-O-tert-butyldimethylsilyl-3'-O-propargyl-α,α,α-trifluorothymidine,
5'-O-di-tert-butylmethylsilyl-3'-O-propargyl-α,α,α-trifluorothymidine,
5'-O-pentyldimethylsilyl-3'-O-propargyl-α,α,α-trifluorothymidine,
5'-O-(2,3-dimethylpropyl)dimethylsilyl-3'-O-propargyl-α,α,α-trifluorothymidine,
5'-O-hexyldimethylsilyl-3'-O-propargyl-α,α,α-trifluorothymidine,
5'-O-trihexylsilyl-3'-O-propargyl-α,α,α-trifluorothymidine,
5'-O-(3,3-dimethylbutyl)dimethyisilyl-3'-O-propargyl-α,α,α-trifluorothymidine,
5'-O-(1,1,2-trimethylpropyl)dimethylsilyl-3'-O-propargyl-α,α,α-trifluorothymidine,
5'-O-heptyldimethylsilyl-3'-O-propargyl-α,α,α-trifluorothymidine,
5'-O-octyldimethylsilyl-3'-O-propargyl-α,α,α-trifluorothymidine,
5'-O-nonyldimethylsilyl-3'-O-propargyl-α,α,α-trifluorothymidine,
5'-O-decyldimethylsilyl-3'-O-propargyl-α,α,α-trifluorothymidine,
5'-O-dodecyldimethylsilyl-3'-O-propargyl-α,α,α-trifluorothymidine,
5'-O-tetradecyldimethylsilyl-3'-O-propargyl-α,α,α-trifluorothymidine,
5'-O-hexadecyldimethylsilyl-3'-O-propargyl-α,α,α-trifluorothymidine,
5'-O-octadecyldimethylsilyl-3'-O-propargyl-α,α,α-trifluorothymidine,
5'-O-eicosyldimethylsilyl-3'-O-propargyl-α,α,α-trifluorothymidine,
5'-O-diphenylmethylsilyl-3'-O-propargyl-α,α,α-trifluorothymidine,
5'-O-phenyldimethylsilyl-3'-O-propargyl-α,α,α-trifluorothymidine,
5'-O-triphenylsilyl-3'-O-propargyl-α,α,α-trifluorothymidine,
5'-O-tert-butyldiphenylsilyl-3'-O-propargyl-α,α,α-trifluorothymidine,
5'-O-diphenylbutylsilyl-3'-O-propargyl-α,α,α-trifluorothymidine,
5'-O-acetyl-3'-O-propargyl-α,α,α-trifluorothymidine,
5'-O-pivaloyl-3'-O-propargyl-α,α,α-trifluorothymidine,
5'-O-methoxymethyl-3'-O-propargyl-α,α,α-trifluorothymidine,
5'-O-benzyl-3,-O-propargyl-α,α,α-trifluorothymidine,
3'-O-propargyl-5,-O-trityl-α,α,α-trifluorothymidine,
3'-O-propargyl-5'-O-butyryl-α,α,α-trifluorothymidine,
3'-O-propargyl-5'-O-valeryl-α,α,α-trifluorothymidine,
3'-O-propargyl-5'-O-hexanoyl-α,α,α-trifluorothymidine,
3'-O-propargyl-5'-O-octanoyl-α,α,α-trifluorothymidine,
3'-O-propargyl-5'-O-nonanoyl-α,α,α-trifluorothymidine,
3'-O-propargyl-5'-O-decanoyl-α,α,α-trifluorothymidine,
3'-O-propargyl-5'-O-lauroyl-α,α,α-trifluorothymidine,
3'-O-propargyl-5'-O-myristoyl-α,α,α-trifluorothymidine,
3'-O-propargyl-5'-O-palmitoyl-α,α,α-trifluorothymidine,
3'-O-propargyl-5'-O-stearoyl-α,α,α-trifluorothymidine,
3'-O-propargyl-5'-O-eicosanoyl-α,α,α-trifluorothymidine,
3'-O-propargyl-5'-O-docosanoyl-α,α,α-trifluorothymidine,
3'-O-propargyl-5'-O-tetracosanoyl-α,α,α-trifluorothymidine,
3'-O-propargyl-5'-O-hexacosanoyl-α,α,α-trifluorothymidine,
3'-O-propargyl-5'-O-octacosanoyl-α,α,α-trifluorothymidine,
3'-O-propargyl-5'-O-crotonoyl-α,α,α-trifluorothymidine,
3'-O-propargyl-5'-O-(9-hexadecenoyl)-α,α,α-trifluorothymidine,
3'-O-propargyl-5'-O-oleoyl-α,α,α-trifluorothymidine,
3'-O-propargyl-5'-O-(11-octadecenoyl)-α,α,α-trifluorothymidine,
3'-O-propargyl-5'-(9,12-octadecadienoyl)-α,α,α-trifluorothymidine,
3'-O-propargyl-5'-O-(9,12,15-octadecatrienoyl)-α,α,α-trifluorothymidine,
3'-O-propargyl-5'-O-(6,9,12-octadecatrienoyl)-α,α,α-trifluorothymidine,
3'-O-propargyl-5'-O-(9,11,13-octadecatrienoyl)-α,α,α-trifluorothymidine,
3'-O-propargyl-5'-O-(8,11-eicosadienoyl)-α,α,α-trifluorothymidine,
3'-O-propargyl-5'-O-(5,8,11-eicosatrienoyl)-α,α,α-trifluorothymidine,
3'-O-propargyl-5'-O-(5,8,11,14-eicosatetraenoyl)-α,α,α-trifluorothymidine,
3'-O-propargyl-5'-O-(15-tetracosaenoyl)-α,α,α-trifluorothymidine,

3'-O-propargyl-5'-O-(4-methylbenzoyl)-α,α,α-trifluorothymidine,
3'-O-propargyl-5'-O-(4-ethylbenzoyl)-α,α,α-trifluorothymidine,
3'-O-propargyl-5'-O-(4-propylbenzoyl)-α,α,α-trifluorothymidine,
3'-O-propargyl-5'-O-(4-butylbenzoyl)-α,α,α-trifluorothymidine,
3'-O-propargyl-5'-O-(4-pentylbenzoyl)-α,α,α-trifluorothymidine,
3'-O-propargyl-5'-O-(4-hexylbenzoyl)-α,α,α-trifluorothymidine,
3'-O-propargyl-5'-O-(4-heptylbenzoyl)-α,α,α-trifluorothymidine,
3'-O-propargyl-5'-O-(4-octylbenzoyl)-α,α,α-trifluorothymidine,
3'-O-propargyl-5'-O-(4-nonylbenzoyl)-α,α,α-trifluorothymidine,
3'-O-propargyl-5'-O-(4-decylbenzoyl)-α,α,α-trifluorothymidine,
3'-O-propargyl-5'-O-methoxycarbonyl-α,α,α-trifluorothymidine,
3'-O-propargyl-5'-O-ethoxycarbonyl-α,α,α-trifluorothymidine,
3'-O-propargyl-5,-O-propoxycarbonyl-α,α,α-trifluorothymidine,
3'-O-propargyl-5'-O-butoxycarbonyl-α,α,α-trifluorothymidine,
3'-O-propargyl-5'-O-pentyloxycarbonyl-α,α,α-trifluorothymidine,
3'-O-propargyl-5'-O-hexyloxycarbonyl-α,α,α-trifluorothymidine,
3'-O-propargyl-5'-O-heptyloxycarbonyl-α,α,α-trifluorothymidine,
3'-O-propargyl-5'-O-octyloxycarbonyl-α,α,α-trifluorothymidine,
3'-O-propargyl-5'-O-nonyloxycarbonyl-α,α,α-trifluorothymidine,
3'-O-propargyl-5'-O-decyloxycarbonyl-α,α,α-trifluorothymidine,
3'-O-propargyl-5'-O-{2-(dimethylamino)ethyloxy}carbonyl-α,α,α-trifluorothymidine,
5'-O-{2-(2-butoxyethoxy)acetyl}-3'-O-propargyl-α,α,α-trifluorothymidine,
5'-O-(N-cyclopentylcarbamoyl)-3'-O-propargyl-α,α,α-trifluorothymidine,
5'-O-(N-cyclohexylcarbamoyl)-3'-O-propargyl-α,α,α-trifluorothymidine,
5'-O-(N-cycloheptylcarbamoyl)-3'-O-propargyl-α,α,α-trifluorothymidine,
5'-O-(N-cyclooctylcarbamoyl)-3'-O-propargyl-α,α,α-trifluorothymidine,
5'-O-(N-cyclopentyl-N-methylcarbamoyl)-3'-O-propargyl-α,α,α-trifluorothymidine,
5'-O-(N-cyclohexyl-N-methylcarbamoyl)-3'-O-propargyl-α,α,α-trifluorothymidine,
5'-O-(N-cycloheptyl-N-methylcarbamoyl)-3'-O-propargyl-α,α,α-trifluorothymidine,
5'-O-(N-cyclooctyl-N-methylcarbamoyl)-3'-O-propargyl-α,α,α-trifluorothymidine,
5'-O-(N-cyclopentyl-N-ethylcarbamoyl)-3'-O-propargyl-α,α,α-trifluorothymidine,
5'-O-(N-cyclohexyl-N-ethylcarbamoyl)-3'-O-propargyl-α,α,α-trifluorothymidine,
5'-O-(N-cycloheptyl-N-ethylcarbamoyl)-3'-O-propargyl-α,α,α-trifluorothymidine,
5'-O-(N-cyclooctyl-N-ethylcarbamoyl)-3'-O-propargyl-α,α,α-trifluorothymidine,
5'-O-(N-cyclopentyl-N-propylcarbamoyl)-3'-O-propargyl-α,α,α-trifluorothymidine,
5'-O-(N-cyclohexyl-N-propylcarbamoyl)-3'-O-propargyl-α,α,α-trifluorothymidine,
5'-O-(N-cycloheptyl-N-propylcarbamoyl)-3'-O-propargyl-α,α,α-trifluorothymidine,
5'-O-(N-cyclooctyl-N-propylcarbamoyl)-3'-O-propargyl-α,α,α-trifluorothymidine,
5'-O-(N-cyclopentyl-N-butylcarbamoyl)-3'-O-propargyl-α,α,α-trifluorothymidine,
5'-O-(N-cyclohexyl-N-butylcarbamoyl)-3'-O-propargyl-α,α,α-trifluorothymidine,
5'-O-(N-cycloheptyl-N-butylcarbamoyl)-3'-O-propargyl-α,α,α-trifluorothymidine,
5'-O-(N-cyclooctyl-N-butylcarbamoyl)-3'-O-propargyl-α,α,α-trifluorothymidine,
5'-O-(N-methylcarbamoyl)-3'-O-propargyl-α,α,α-trifluorothymidine,
5'-O-(N-ethylcarbamoyl)-3'-O-propargyl-α,α,α-trifluorothymidine,
5'-O-(N-propylcarbamoyl)-3'-O-propargyl-α,α,α-trifluorothymidine,
5'-O-(N-butylcarbamoyl)-3'-O-propargyl-α,α,α-trifluorothymidine,
5'-O-(N-pentylcarbamoyl)-3'-O-propargyl-α,α,α-trifluorothymidine,
5'-O-(N-hexylcarbamoyl)-3'-O-propargyl-α,α,α-trifluorothymidine,
5'-O-(N-heptylcarbamoyl)-3'-O-propargyl-α,α,α-trifluorothymidine,
5'-O-(N-octylcarbamoyl)-3'-O-propargyl-α,α,α-trifluorothymidine,
5'-O-(N-nonylcarbamoyl)-3'-O-propargyl-α,α,α-trifluorothymidine,
5'-O-(N-dodecylcarbamoyl)-3,-O-propargyl-α,α,α-trifluorothymidine,
5'-O-(N-tetradecylcarbamoyl)-3'-O-propargyl-α,α,α-trifluorothymidine,
5'-O-(N-hexadecylcarbamoyl)-3'-O-propargyl-α,α,α-trifluorothymidine,
5'-O-(N-octadecylcarbamoyl)-3'-O-propargyl-α,α,α-trifluorothymidine,
5'-O-(N-eicosanylcarbamoyl)-3'-O-propargyl-α,α,α-trifluorothymidine,
5'-O-(N-docosanylcarbamoyl)-3'-O-propargyl-α,α,α-trifluorothymidine,
5'-O-(N-tetracosanylcarbamoyl)-3'-O-propargyl-α,α,α-trifluorothymidine,

5'-O-(N-hexacosanylcarbamoyl)-3'-O-propargyl-α,α,α-trifluorothymidine,
5'-O-(N-octacosanylcarbamoyl)-3'-O-propargyl-α,α,α-trifluorothymidine,
5'-O-(N-triancotanylcarbamoyl)-3'-O-propargyl-α,α,α-trifluorothymidine,
5'-O-(N,N-dimethylcarbamoyl)-3'-O-propargyl-α,α,α-trifluorothymidine,
5'-O-(N-ethyl-N-methylcarbamoyl)-3'-O-propargyl-α,α,α-trifluorothymidine,
5'-O-(N-propyl-N-methylcarbamoyl)-3'-O-propargyl-α,α,α-trifluorothymidine,
5'-O-(N-butyl-N-methylcarbamoyl)-3'-O-propargyl-α,α,α-trifluorothymidine,
5'-O-(N-pentyl-N-methylcarbamoyl)-3'-O-propargyl-α,α,α-trifluorothymidine,
5'-O-(N-hexyl-N-methylcarbamoyl)-3'-O-propargyl-α,α,α-trifluorothymidine,
5'-O-(N-heptyl-N-methylcarbamoyl)-3'-O-propargyl-α,α,α-trifluorothymidine,
5'-O-(N-octyl-N-methylcarbamoyl)-3'-O-propargyl-α,α,α-trifluorothymidine,
5'-O-(N-nonyl-N-methylcarbamoyl)-3'-O-propargyl-α,α,α-trifluorothymidine,
5'-O-(N-dodecyl-N-methylcarbamoyl)-3'-O-propargyl-α,α,α-trifluorothymidine,
5'-O-(N-tetradecyl-N-methylcarbamoyl)-3'-O-propargyl-α,α,α-trifluorothymidine,
5'-O-(N-hexadecyl-N-methylcarbamoyl)-3'-O-propargyl-α,α,α-trifluorothymidine,
5'-O-(N-octadecyl-N-methylcarbamoyl)-3'-O-propargyl-α,α,α-trifluorothymidine,
5'-O-(N,N-diethylcarbamoyl)-3'-O-propargyl-α,α,α-trifluorothymidine,
5'-O-(N-propyl-N-ethylcarbamoyl)-3'-O-propargyl-α,α,α-trifluorothymidine,
5'-O-(N-butyl-N-ethylcarbamoyl)-3'-O-propargyl-α,α,α-trifluorothymidine,
5'-O-(N-pentyl-N-ethylcarbamoyl)-3'-O-propargyl-α,α,α-trifluorothymidine,
5'-O-(N-hexyl-N-ethylcarbamoyl)-3'-O-propargyl-α,α,α-trifluorothymidine,
5'-O-(N-heptyl-N-ethylcarbamoyl)-3'-O-propargyl-α,α,α-trifluorothymidine,
5'-O-(N-octyl-N-ethylcarbamoyl)-3'-O-propargyl-α,α,α-trifluorothymidine,
5'-O-(N-nonyl-N-ethylcarbamoyl)-3'-O-propargyl-α,α,α-trifluorothymidine,
5'-O-(N-dodecyl-N-ethylcarbamoyl)-3'-O-propargyl-α,α,α-trifluorothymidine,
5'-O-(N-tetradecyl-N-ethylcarbamoyl)-3'-O-propargyl-α,α,α-trifluorothymidine,
5'-O-(N-hexadecyl-N-ethylcarbamoyl)-3'-O-propargyl-α,α,α-trifluorothymidine,
5'-O-(N-octadecyl-N-ethylcarbamoyl)-3'-O-propargyl-α,α,α-trifluorothymidine,
5'-O-(N,N-dipropylcarbamoyl)-3'-O-propargyl-α,α,α-trifluorothymidine,
5'-O-(N-butyl-N-propylcarbamoyl)-3'-O-propargyl-α,α,α-trifluorothymidine,
5'-O-(N-pentyl-N-propylcarbamoyl)-3'-O-propargyl-α,α,α-trifluorothymidine,
5'-O-(N-hexyl-N-propylcarbamoyl)-3'-O-propargyl-α,α,α-trifluorothymidine,
5'-O-(N-heptyl-N-propylcarbamoyl)-3'-O-propargyl-α,α,α-trifluorothymidine,
5'-O-(N-octyl-N-propylcarbamoyl)-3'-O-propargyl-α,α,α-trifluorothymidine,
5'-O-(N-nonyl-N-propylcarbamoyl)-3'-O-propargyl-α,α,α-trifluorothymidine,
5'-O-(N-dodecyl-N-propylcarbamoyl)-3'-O-propargyl-α,α,α-trifluorothymidine,
5'-O-(N-tetradecyl-N-propylcarbamoyl)-3'-O-propargyl-α,α,α-trifluorothymidine,
'-O-(N-hexadecyl-N-propylcarbamoyl)-3'-O-propargyl-α,α,α-trifluorothymidine,
5'-O-(N-octadecyl-N-propylcarbamoyl)-3'-O-propargyl-α,α,α-trifluorothymidine,
5'-O-(N,N-dibutylcarbamoyl)-3'-O-propargyl-α,α,α-trifluorothymidine,
5'-O-(N-pentyl-N-butylcarbamoyl)-3'-O-propargyl-α,α,α-trifluorothymidine,
5'-O-(N-hexyl-N-butylcarbamoyl)-3'-O-propargyl-α,α,α-trifluorothymidine,
5'-O-(N-heptyl-N-butylcarbamoyl)-3'-O-propargyl-α,α,α-trifluorothymidine,
5'-O-(N-octyl-N-butylcarbamoyl)-3'-O-propargyl-α,α,α-trifluorothymidine,
5'-O-(N-nonyl-N-butylcarbamoyl)-3'-O-propargyl-α,α,α-trifluorothymidine,
5'-O-(N-dodecyl-N-butylcarbamoyl)-3'-O-propargyl-α,α,α-trifluorothymidine,
5'-O-(N-tetradecyl-N-butylcarbamoyl)-3'-O-propargyl-α,α,α-trifluorothymidine,
5'-O-(N-hexadecyl-N-butylcarbamoyl)-3'-O-propargyl-α,α,α-trifluorothymidine,
5'-O-(N-octadecyl-N-butylcarbamoyl)-3'-O-propargyl-α,α,α-trifluorothymidine,
5'-O-[N-{3-(diethylamino)propyl}carbamoyl}-3'-O-propargyl-α,α,α-trifluorothymidine,
5'-O-{2-(4-methylpiperazin-1-yl)acetyl}-3'-O-propargyl-α,α,α-trifluorothymidine,
5'-O-{2-(4-ethylpiperazin-1-yl)acetyl}-3'-O-propargyl-α,α,α-trifluorothymidine,
5'-O-{2-(4-propylpiperazin-1-yl)acetyl}-3'-O-propargyl-α,α,α-trifluorothymidine,
5'-O-{2-(4-butylpiperazin-1-yl)acetyl}-3'-O-propargyl-α,α,α-trifluorothymidine,
5'-O-{2-(4-pentylpiperazin-1-yl)acetyl}-3'-O-propargyl-α,α,α-trifluorothymidine,
5'-O-{2-(4-hexylpiperazin-1-yl)acetyl}-3,-O-propargyl-α,α,α-trifluorothymidine,
5'-O-{2-(4-heptylpiperazin-1-yl)acetyl}-3'-O-propargyl-α,α,α-trifluorothymidine,
5'-O-{2-(4-octylpiperazin-1-yl)acetyl}-3'-O-propargyl-α,α,α-trifluorothymidine,

5'-O-{2-(4-nonylpiperazin-1-yl)acetyl}-3'-O-propargyl-α,α,α-trifluorothymidine,
5'-O-{2-(4-decylpiperazin-1-yl)acetyl}-3'-O-propargyl-α,α,α-trifluorothymidine,
5'-O-(N-methyl-L-prolyl)-3'-O-propargyl-α,α,α-trifluorothymidine,
5'-O-(N-ethyl-L-prolyl)-3'-O-propargyl-α,α,α-trifluorothymidine,
5'-O-(N-propyl-L-prolyl)-3'-O-propargyl-α,α,α-trifluorothymidine,
5'-O-(N-butyl-L-prolyl)-3'-O-propargyl-α,α,α-trifluorothymidine,
5'-O-(N-pentyl-L-prolyl)-3'-O-propargyl-α,α,α-trifluorothymidine,
5'-O-(N-hexyl-L-prolyl)-3'-O-propargyl-α,α,α-trifluorothymidine,
5'-O-(N-heptyl-L-prolyl)-3'-O-propargyl-α,α,α-trifluorothymidine,
5'-O-(N-octyl-L-prolyl)-3'-O-propargyl-α,α,α-trifluorothymidine,
5'-O-(N-nonyl-L-prolyl)-3,-O-propargyl-α,α,α-trifluorothymidine,
5'-O-(N-decyl-L-prolyl)-3'-O-propargyl-α,α,α-trifluorothymidine,
5'-O-(N-dodecyl-L-prolyl)-3'-O-propargyl-α,α,α-trifluorothymidine,
5'-O-(N-tetradecyl-L-prolyl)-3'-O-propargyl-α,α,α-trifluorothymidine,
5'-O-(N-hexadecyl-L-prolyl)-3'-O-propargyl-α,α,α-trifluorothymidine,
5'-O-(N-octadecyl-L-prolyl)-3'-O-propargyl-α,α,α-trifluorothymidine,
5'-O-valyl-3'-O-propargyl-α,α,α-trifluorothymidine,
5'-O-(ethoxyhydroxyphosphoryl)-3'-O-propargyl-α,α,α-trifluorothymidine,
5'-O-(butoxyhydroxyphosphoryl)-3'-O-propargyl-α,α,α-trifluorothymidine,
5'-O-(hexyloxyhydroxyphosphoryl)-3'-O-propargyl-α,α,α-trifluorothymidine,
5'-O-(octyloxyhydroxyphosphoryl)-3'-O-propargyl-α,α,α-trifluorothymidine,
5'-O-(decyloxyhydroxyphosphoryl)-3'-O-propargyl-α,α,α-trifluorothymidine,
5'-O-(dodecyloxyhydroxyphosphoryl)-3'-O-propargyl-α,α,α-trifluorothymidine,
5'-O-dibutoxyphosphoryl-3'-O-propargyl-α,α,α-trifluorothymidine,
5'-O-(2-oxo-1,3-dioxaphospholan-2-yl)-3'-O-propargyl-α,α,α-trifluorothymidine,
3'-O-propargyl-3-benzoyl-α,α,α-trifluorothymidine,
5'-O-t-butyldimethylsilyl-3'-O-propargyl-3-benzoyl-α,α,α-trifluorothymidine,
5'-O-acetyl-3'-O-propargyl-3-benzoyl-α,α,α-trifluorothymidine,
5'-O-pivaloyl-3'-O-propargyl-3-benzoyl-α,α,α-trifluorothymidine,
5'-O-methoxymethyl-3'-O-propargyl-3-benzoyl-α,α,α-trifluorothymidine,
5'-O-benzyl-3'-O-propargyl-3-benzoyl-α,α,α-trifluorothymidine,
3'-O-propargyl-5'-O-trityl-3-benzoyl-α,α,α-trifluorothymidine,
3'-O-propargyl-3-(4-methoxybenzoyl)-α,α,α-trifluorothymidine,
5'-O-t-butyldimethylsilyl-3'-O-propargyl-3-(4-methoxybenzoyl)-α,α,α-trifluorothymidine,
5'-O-acetyl-3'-O-propargyl-3-(4-methoxybenzoyl)-α,α,α-trifluorothymidine,
5'-O-pivaloyl-3'-O-propargyl-3-(4-methoxybenzoyl)-α,α,α-trifluorothymidine,
5'-O-methoxymethyl-3'-O-propargyl-3-(4-methoxybenzoyl)-α,α,α-trifluorothymidine,
5'-O-benzyl-3'-O-propargyl-3-(4-methoxybenzoyl)-α,α,α-trifluorothymidine,
3'-O-propargyl-5'-O-trityl-3-(4-methoxybenzoyl)-α,α,α-trifluorothymidine,
3'-O-propargyl-3-(2-furoyl)-α,α,α-trifluorothymidine,
5'-O-t-butyldimethylsilyl-3'-O-propargyl-3-(2-furoyl)-α,α,α-trifluorothymidine,
5'-O-acetyl-3'-O-propargyl-3-(2-furoyl)-α,α,α-trifluorothymidine,
5'-O-pivaloyl-3'-O-propargyl-3-(2-furoyl)-α,α,α-trifluorothymidine,
5'-O-methoxymethyl-3'-O-propargyl-3-(2-furoyl)-α,α,α-trifluorothymidine,
5'-O-benzyl-3'-O-propargyl-3-(2-furoyl)-α,α,α-trifluorothymidine,
3'-O-propargyl-5'-O-trityl-3-(2-furoyl)-α,α,α-trifluorothymidine,
3'-O-propargyl-3-acetyl-α,α,α-trifluorothymidine,
5'-O-t-butyldimethylsilyl-3'-O-propargyl-3-acetyl-α,α,α-trifluorothymidine,
3-N:5'-O-diacetyl-3'-O-propargyl-α,α,α-trifluorothymidine,
5'-O-pivaloyl-3'-O-propargyl-3-acetyl-α,α,α-trifluorothymidine,
5'-O-methoxymethyl-3'-O-propargyl-3-acetyl-α,α,α-trifluorothymidine,
5'-O-benzyl-3'-O-propargyl-3-acetyl-α,α,α-trifluorothymidine,
3'-O-propargyl-5'-O-trityl-3-acetyl-α,α,α-trifluorothymidine,
3'-O-propargyl-3-pivaloyl-α,α,α-trifluorothymidine,
5'-O-t-butyldimethylsilyl-3'-O-propargyl-3-pivaloyl-α,α,α-trifluorothymidine,
5'-O-acetyl-3'-O-propargyl-3-pivaloyl-α,α,α-trifluorothymidine,
3-N:5'-O-dipivaloyl-3'-O-propargyl-α,α,α-trifluorothymidine,
5'-O-methoxymethyl-3'-O-propargyl-3-pivaloyl-α,α,α-trifluorothymidine,

5'-O-benzyl-3'-O-propargyl-3-pivaloyl-α,α,α-trifluorothymidine,
3'-O-propargyl-5'-O-trityl-3-pivaloyl-α,α,α-trifluorothymidine,
3'-O-(2-butynyl)-α,α,α-trifluorothymidine,
5'-O-t-butyldimethylsilyl-3'-O-(2-butynyl)-α,α,α-trifluorothymidine,
5'-O-acetyl-3'-O-(2-butynyl)-α,α,α-trifluorothymidine,
3'-O-(2-butynyl)-5'-O-pivaloyl-α,α,α-trifluorothymidine,
3'-O-(2-butynyl)-5'-O-methoxymethyl-α,α,α-trifluorothymidine,
5'-O-benzyl-3'-O-(2-butynyl)-α,α,α-trifluorothymidine,
3'-O-(2-butynyl)-5'-O-trityl-α,α,α-trifluorothymidine,
3'-O-(2-butynyl)-3-benzoyl-α,α,α-trifluorothymidine,
5'-O-t-butyldimethylsilyl-3'-O-(2-butynyl)-3-benzoyl-α,α,α-trifluorothymidine,
5'-O-acetyl-3'-O-(2-butynyl)-3-benzoyl-α,α,α-trifluorothymidine,
3'-O-(2-butynyl)-5'-O-pivaloyl-3-benzoyl-α,α,α-trifluorothymidine,
3'-O-(2-butynyl)-5'-O-methoxymethyl-3-benzoyl-α,α,α-trifluorothymidine,
3'-O-(2-butynyl)-3-(4-methoxybenzoyl)-α,α,α-trifluorothymidine,
5'-O-t-butyldimethylsilyl-3'-O-(2-butynyl)-3-(4-methoxybenzoyl)-α,α,α-trifluorothymidine,
5'-O-acetyl-3'-O-(2-butynyl)-3-(4-methoxybenzoyl)-α,α,α-trifluorothymidine,
3'-O-(2-butynyl)-5'-O-pivaloyl-3-(4-methoxybenzoyl)-α,α,α-trifluorothymidine,
3'-O-(2-butynyl)-5'-O-methoxymethyl-3-(4-methoxybenzoyl)-α,α,α-trifluorothymidine,
3'-O-(2-butynyl)-3-(2-furoyl)-α,α,α-trifluorothymidine,
5'-O-t-butyldimethylsilyl-3'-O-(2-butynyl)-3-(2-furoyl)-α,α,α-trifluorothymidine,
5'-O-acetyl-3'-O-(2-butynyl)-3-(2-furoyl)-α,α,α-trifluorothymidine,
3'-O-(2-butynyl)-5'-O-pivaloyl-3-(2-furoyl)-α,α,α-trifluorothymidine,
3'-O-(2-butynyl)-5'-O-methoxymethyl-3-(2-furoyl)-α,α,α-trifluorothymidine,
3'-O-(2-pentynyl)-α,α,α-trifluorothymidine,
5'-O-t-butyldimethylsilyl-3'-O-(2-pentynyl)-α,α,α-trifluorothymidine,
5'-O-acetyl-3'-O-(2-pentynyl)-α,α,α-trifluorothymidine,
3'-O-(2-pentynyl)-5'-O-pivaloyl-3-α,α,α-trifluorothymidine,
5'-O-methoxymethyl-3'-O-(2-pentynyl)-α,α,α-trifluorothymidine,
5'-O-benzyl-3'-O-(2-pentynyl)-α,α,α-trifluorothymidine,
3'-O-(2-pentynyl)-5'-O-trityl-α,α,α-trifluorothymidine,
3'-O-(2-pentynyl)-3-benzoyl-α,α,α-trifluorothymidine,
5'-O-t-butyldimethylsilyl-3'-O-(2-pentynyl)-3-benzoyl-α,α,α-trifluorothymidine,
5'-O-acetyl-3'-O-(2-pentynyl)-3-benzoyl-α,α,α-trifluorothymidine,
3'-O-(2-pentynyl)-5'-O-pivaloyl-3-benzoyl-α,α,α-trifluorothymidine,
5'-O-methoxymethyl-3'-O-(2-pentynyl)-3-benzoyl-α,α,α-trifluorothymidine,
3'-O-(2-pentynyl)-3-(4-methoxybenzoyl)-α,α,α-trifluorothymidine,
5'-O-t-butyldimethylsilyl-3'-O-(2-pentynyl)-3-(4-methoxybenzoyl)-α,α,α-trifluorothymidine,
5'-O-acetyl-3'-O-(2-pentynyl)-3-(4-methoxybenzoyl)-α,α,α-trifluorothymidine,
3'-O-(2-pentynyl)-5'-O-pivaloyl-3-(4-methoxybenzoyl)-α,α,α-trifluorothymidine,
5'-O-methoxymethyl-3'-O-(2-pentynyl)-3-(4-methoxybenzoyl)-α,α,α-trifluorothymidine,
3'-O-(2-pentynyl)-3-(2-furoyl)-α,α,α-trifluorothymidine,
5'-O-t-butyldimethylsilyl-3'-O-(2-pentynyl)-3-(2-furoyl)-α,α,α-trifluorothymidine,
5'-O-acetyl-3'-O-(2-pentynyl)-3-(2-furoyl)-α,α,α-trifluorothymidine,
3'-O-(2-pentynyl)-5'-O-pivaloyl-3-(2-furoyl)-α,α,α-trifluorothymidine,
5'-O-methoxymethyl-3'-O-(2-pentynyl)-3-(2-furoyl)-α,α,α-trifluorothymidine,
3'-O-(2-hexynyl)-α,α,α-trifluorothymidine,
5,-O-t-butyldimethylsilyl-3'-O-(2-hexynyl)-α,α,α-trifluorothymidine,
5'-O-acetyl-3'-O-(2-hexynyl)-α,α,α-trifluorothymidine,
3'-O-(2-hexynyl)-5'-O-pivaloyl-α,α,α-trifluorothymidine,
3'-O-(2-hexynyl)-5'-O-methoxymethyl-α,α,α-trifluorothymidine,
5'-O-benzyl-3'-O-(2-hexynyl)-α,α,α-trifluorothymidine,
3'-O-(2-hexynyl)-5'-O-trityl-α,α,α-trifluorothymidine,
3'-O-(2-hexynyl)-3-benzoyl-α,α,α-trifluorothymidine,
5'-O-t-butyldimethylsilyl-3'-O-(2-hexynyl)-3-benzoyl-α,α,α-trifluorothymidine,
5'-O-acetyl-3'-O-(2-hexynyl)-3-benzoyl-α,α,α-trifluorothymidine,
3'-O-(2-hexynyl)-5'-O-pivaloyl-3-benzoyl-α,α,α-trifluorothymidine,
3'-O-(2-hexynyl)-5'-O-methoxymethyl-3-benzoyl-α,α,α-trifluorothymidine,

3'-O-(2-hexynyl)-3-(4-methoxybenzoyl)-α,α,α-trifluorothymidine,
5'-O-t-butyldimethylsilyl-3'-O-(2-hexynyl)-3-(4-methoxybenzoyl)-α,α,α-trifluorothymidine,
5'-O-acetyl-3'-O-(2-hexynyl)-3-(4-methoxybenzoyl)-α,α,α-trifluorothymidine,
3'-O-(2-hexynyl)-5'-O-pivaloyl-3-(4-methoxybenzoyl)-α,α,α-trifluorothymidine,
3'-O-(2-hexynyl)-5'-O-methoxymethyl-3-(4-methoxybenzoyl)-α,α,α-trifluorothymidine,
3'-O-(2-hexynyl)-3-(2-furoyl)-α,α,α-trifluorothymidine,
5'-O-t-butyldimethylsilyl-3'-O-(2-hexynyl)-3-(2-furoyl)-α,α,α-trifluorothymidine,
5'-O-acetyl-3'-O-(2-hexynyl)-3-(2-furoyl)-α,α,α-trifluorothymidine,
3'-O-(2-hexynyl)-5'-O-pivaloyl-3-(2-furoyl)-α,α,α-trifluorothymidine,
3'-O-(2-hexynyl)-5'-O-methoxymethyl-3-(2-furoyl)-α,α,α-trifluorothymidine,
3'-O-(2-heptynyl)-α,α,α-trifluorothymidine,
5'-O-t-butyldimethylsilyl-3'-O-(2-heptynyl)-α,α,α-trifluorothymidine,
5'-O-acetyl-3'-O-(2-heptynyl)-α,α,α-trifluorothymidine,
3'-O-(2-heptynyl)-5'-O-pivaloyl-α,α,α-trifluorothymidine,
3'-O-(2-heptynyl)-5'-O-methoxymethyl-α,α,α-trifluorothymidine,
5'-O-benzyl-3'-O-(2-heptynyl)-α,α,α-trifluorothymidine,
3'-O-(2-heptynyl)-5'-O-trityl-α,α,α-trifluorothymidine,
3'-O-(2-heptynyl)-3-benzoyl-α,α,α-trifluozothymidine,
5'-O-t-butyldimethylsilyl-3'-O-(2-heptynyl)-3-benzoyl-α,α,α-trifluorothymidine,
5'-O-acetyl-3'-O-(2-heptynyl)-3-benzoyl-α,α,α-trifluorothymidine,
3'-O-(2-heptynyl)-5'-O-pivaloyl-3-benzoyl-α,α,α-trifluorothymidine,
3'-O-(2-heptynyl)-5'-O-methoxymethyl-3-benzoyl-α,α,α-trifluorothymidine,
3'-O-(2-heptynyl)-3-(4-methoxybenzoyl)-α,α,α-trifluorothymidine,
5'-O-t-butyldimethylsilyl-3'-O-(2-heptynyl)-3-(4-methoxybenzoyl)-α,α,α-trifluorothymidine,
5'-O-acetyl-3'-O-(2-heptynyl)-3-(4-methoxybenzoyl)-α,α,α-trifluorothymidine,
3'-O-(2-heptynyl)-5'-O-pivaloyl-3-(4-methoxybenzoyl)-α,α,α-trifluorothymidine,
3'-O-(2-heptynyl)-5'-O-methoxymethyl-3-(4-methoxybenzoyl)-α,α,α-trifluorothymidine,
3'-O-(2-heptynyl)-3-(2-furoyl)-α,α,α-trifluorothymidine,
5'-O-t-butyldimethylsilyl-3'-O-(2-heptynyl)-3-(2-furoyl)-α,α,α-trifluorothymidine,
5'-O-acetyl-3'-O-(2-heptynyl)-3-(2-furoyl)-α,α,α-trifluorothymidine,
5'-O-pivaloyl-3'-O-(2-heptynyl)-3-(2-furoyl)-α,α,α-trifluorothymidine,
3'-O-(2-heptynyl)-5'-O-methoxymethyl-3-(2-furoyl)-α,α,α-trifluorothymidine,
3'-O-(4-methyl-2-pentynyl)-α,α,α-trifluorothymidine,
5'-O-t-butyldimethylsilyl-3'-O-(4-methyl-2-pentynyl)-α,α,α-trifluorothymidine,
5'-O-acetyl-3'-O-(4-methyl-2-pentynyl)-α,α,α-trifluorothymidine,
3'-O-(4-methyl-2-pentynyl)-5'-O-pivaloyl-3-α,α,α-trifluorothymidine,
5'-O-methoxymethyl-3'-O-(4-methyl-2-pentynyl)-α,α,α-trifluorothymidine,
5'-O-benzyl-3'-O-(4-methyl-2-pentynyl)-α,α,α-trifluorothymidine,
3'-O-(4-methyl-2-pentynyl)-5'-O-trityl-α,α,α-trifluorothymidine,
3'-O-(4-methyl-2-pentynyl)-3-benzoyl-α,α,α-trifluorothymidine,
5'-O-t-butyldimethylsilyl-3'-O-(4-methyl-2-pentynyl)-3-benzoyl-α,α,α-trifluorothymidine,
5'-O-acetyl-3'-O-(4-methyl-2-pentynyl)-3-benzoyl-α,α,α-trifluorothymidine,
3'-O-(4-methyl-2-pentynyl)-5'-O-pivaloyl-3-benzoyl-α,α,α-trifluorothymidine,
5'-O-methoxymethyl-3'-O-(4-methyl-2-pentynyl)-3-benzoyl-α,α,α-trifluorothymidine,
3'-O-(4-methyl-2-pentynyl)-3-(4-methoxybenzoyl)-α,α,α-trifluorothymidine,
5'-O-t-butyldimethylsilyl-3'-O-(4-methyl-2-pentynyl)-3-(4-methoxybenzoyl)-α,α,α-trifluorothymidine,
5'-O-acetyl-3'-O-(4-methyl-2-pentynyl)-3-(4-methoxybenzoyl)-α,α,α-trifluorothymidine,
3'-O-(4-methyl-2-pentynyl)-5'-O-pivaloyl-3-(4-methoxybenzoyl)-α,α,α-trifluorothymidine,
5'-O-methoxymethyl-3'-O-(4-methyl-2-pentynyl)-3-(4-methoxybenzoyl)-α,α,α-trifluorothymidine,
3'-O-(4-methyl-2-pentynyl)-3-(2-furoyl)-α,α,α-trifluorothymidine,
5'-O-t-butyldimethylsilyl-3'-O-(4-methyl-2-pentynyl)-3-(2-furoyl)-α,α,α-trifluorothymidine,
5'-O-acetyl-3'-O-(4-methyl-2-pentynyl)-3-(2-furoyl)-α,α,α-trifluorothymidine,
3'-O-(4-methyl-2-pentynyl)-5'-O-pivaloyl-3-(2-furoyl)-α,α,α-trifluorothymidine,
5'-O-methoxymethyl-3'-O-(4-methyl-2-pentynyl)-3-(2-furoyl)-α,α,α-trifluorothymidine,
3'-O-(4,4-dimethyl-2-pentynyl)-α,α,α-trifluorothymidine,
5'-O-t-butyldimethylsilyl-3'-O-(4,4-dimethyl-2-pentynyl)-α,α,α-trifluorothymidine,
5'-O-acetyl-3'-O-(4,4-dimethyl-2-pentynyl)-α,α,α-trifluorothymidine,
3'-O-(4,4-dimethyl-2-pentynyl)-5'-O-pivaloyl-α,α,α-trifluorothymidine,

3'-O-(4,4-dimethyl-2-pentynyl)-5'-O-methoxymethyl-$\alpha,\alpha,\alpha$-trifluorothymidine,
5'-O-benzyl-3'-O-(4,4-dimethyl-2-pentynyl)-$\alpha,\alpha,\alpha$-trifluorothymidine,
3'-O-(4,4-dimethyl-2-pentynyl)-5'-O-trityl-$\alpha,\alpha,\alpha$-trifluorothymidine,
3'-O-(4,4-dimethyl-2-pentynyl)-3-benzoyl-$\alpha,\alpha,\alpha$-trifluorothymidine,
5'-O-t-butyldimethylsilyl-3'-O-(4,4-dimethyl-2-pentynyl)-3-benzoyl-$\alpha,\alpha,\alpha$-trifluorothymidine,
5'-O-acetyl-3'-O-(4,4-dimethyl-2-pentynyl)-3-benzoyl-$\alpha,\alpha,\alpha$-trifluorothymidine,
3'-O-(4,4-dimethyl-2-pentynyl)-5'-O-pivaloyl-3-benzoyl-$\alpha,\alpha,\alpha$-trifluorothymidine,
3'-O-(4,4-dimethyl-2-pentynyl)-5'-O-methoxymethyl-3-benzoyl-$\alpha,\alpha,\alpha$-trifluorothymidine,
3'-O-(4,4-dimethyl-2-pentynyl)-3-(4-methoxybenzoyl)-$\alpha,\alpha,\alpha$-trifluorothymidine,
5'-O-t-butyldimethylsilyl-3'-O-(4,4-dimethyl-2-pentynyl)-3-(4-methoxybenzoyl)-$\alpha,\alpha,\alpha$-trifluorothymidine,
5'-O-acetyl-3'-O-(4,4-dimethyl-2-pentynyl)-3-(4-methoxybenzoyl)-$\alpha,\alpha,\alpha$-trifluorothymidine,
3'-O-(4,4-dimethyl-2-pentynyl)-5'-O-pivaloyl-3-(4-methoxybenzoyl)-$\alpha,\alpha,\alpha$-trifluorothymidine,
3'-O-(4,4-dimethyl-2-pentynyl)-5'-O-methoxymethyl-3-(4-methoxybenzoyl)-$\alpha,\alpha,\alpha$-trifluorothymidine,
3'-O-(4,4-dimethyl-2-pentynyl)-3-(2-furoyl)-$\alpha,\alpha,\alpha$-trifluorothymidine,
5'-O-t-butyldimethylsilyl-3'-O-(4,4-dimethyl-2-pentynyl)-3-(2-furoyl)-$\alpha,\alpha,\alpha$-trifluorothymidine,
5'-O-acetyl-3'-O-(4,4-dimethyl-2-pentynyl)-3-(2-furoyl)-$\alpha,\alpha,\alpha$-trifluorothymidine,
3'-O-(4,4-dimethyl-2-pentynyl)-5'-O-pivaloyl-3-(2-furoyl)-$\alpha,\alpha,\alpha$-trifluorothymidine,
3'-O-(4,4-dimethyl-2-pentynyl)-5'-O-methoxymethyl-3-(2-furoyl)-$\alpha,\alpha,\alpha$-trifluorothymidine,
3'-O-(5-methyl-2-hexynyl)-$\alpha,\alpha,\alpha$-trifluorothymidine,
5'-O-t-butyldimethylsilyl-3'-O-(5-methyl-2-hexynyl)-3-benzoyl-$\alpha,\alpha,\alpha$-trifluorothymidine,
5'-O-acetyl-3'-O-(5-methyl-2-hexynyl)-$\alpha,\alpha,\alpha$-trifluorothymidine,
3'-O-(5-methyl-2-hexynyl)-5'-O-pivaloyl-$\alpha,\alpha,\alpha$-trifluorothymidine,
5'-O-methoxymethyl-3'-O-(5-methyl-2-hexynyl)-$\alpha,\alpha,\alpha$-trifluorothymidine,
5'-O-benzyl-3'-O-(5-methyl-2-hexynyl)-$\alpha,\alpha,\alpha$-trifluorothymidine,
3'-O-(5-methyl-2-hexynyl)-5'-O-trityl-$\alpha,\alpha,\alpha$-trifluorothymidine,
3'-O-(5-methyl-2-hexynyl)-3-benzoyl-$\alpha,\alpha,\alpha$-trifluorothymidine,
5'-O-t-butyldimethylsilyl-3'-O-(5-methyl-2-hexynyl)-3-benzoyl-$\alpha,\alpha,\alpha$-trifluorothymidine,
5'-O-acetyl-3'-O-(5-methyl-2-hexynyl)-3-benzoyl-$\alpha,\alpha,\alpha$-trifluorothymidine,
3'-O-(5-methyl-2-hexynyl)-5'-O-pivaloyl-3-benzoyl-$\alpha,\alpha,\alpha$-trifluorothymidine,
5'-O-methoxymethyl-3'-O-(5-methyl-2-hexynyl)-3-benzoyl-$\alpha,\alpha,\alpha$-trifluorothymidine,
3'-O-(5-methyl-2-hexynyl)-3-(4-methoxybenzoyl)-$\alpha,\alpha,\alpha$-trifluorothymidine,
5'-O-t-butyldimethylsilyl-3'-O-(5-methyl-2-hexynyl)-3-(4-methoxybenzoyl)-$\alpha,\alpha,\alpha$-trifluorothymidine,
5'-O-acetyl-3'-O-(5-methyl-2-hexynyl)-3-(4-methoxybenzoyl)-$\alpha,\alpha,\alpha$-trifluorothymidine,
3'-O-(5-methyl-2-hexynyl)-5'-O-pivaloyl-3-(4-methoxybenzoyl)-$\alpha,\alpha,\alpha$-trifluorothymidine,
5'-O-methoxymethyl-3'-O-(5-methyl-2-hexynyl)-3-(4-methoxybenzoyl)-$\alpha,\alpha,\alpha$-trifluorothymidine,
3'-O-(5-methyl-2-hexynyl)-3-(2-furoyl)-$\alpha,\alpha,\alpha$-trifluorothymidine,
5'-O-t-butyldimethylsilyl-3'-O-(5-methyl-2-hexynyl)-3-(2-furoyl)-$\alpha,\alpha,\alpha$-trifluorothymidine,
5'-O-acetyl-3'-O-(5-methyl-2-hexynyl)-3-(2-furoyl)-$\alpha,\alpha,\alpha$-trifluorothymidine,
5'-O-pivaloyl-3'-O-(5-methyl-2-hexynyl)-3-(2-furoyl)-$\alpha,\alpha,\alpha$-trifluorothymidine, and
5'-O-methoxymethyl-3'-O-(5-methyl-2-hexynyl)-3-(2-furoyl)-$\alpha,\alpha,\alpha$-trifluorothymidine.

Among the compounds represented by the formula [I], those represented by the formula [Ia] may be prepared by reacting F3Thd or a F3Thd derivative in which the 5'-position is protected by a protective group (i.e., the compound represented by the formula [III]) with a compound of the formula [IV] in an appropriate solvent in the presence of a base, according to the following reaction equation:

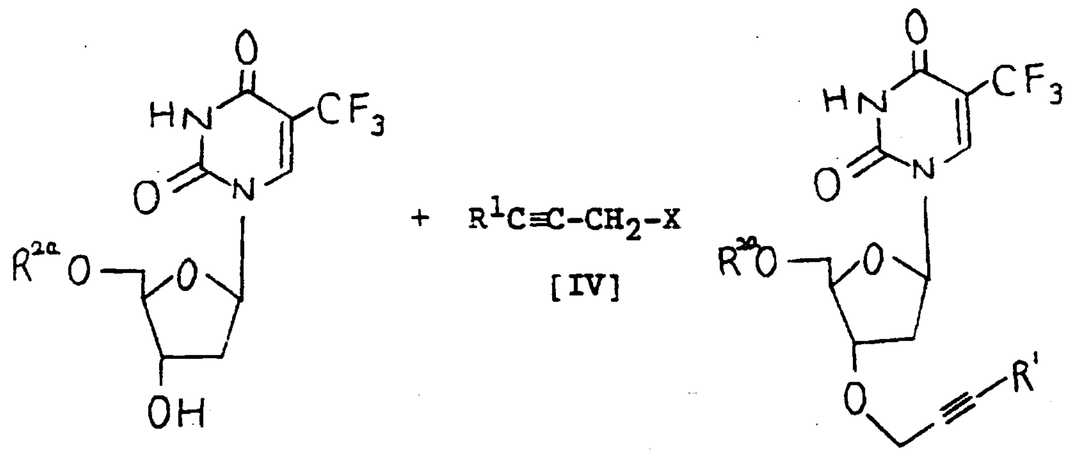

+ $R^1C{\equiv}C\text{-}CH_2\text{-}X$

[IV]

[III] [Ia]

(wherein in formula [III], $R^1$ represents the same meaning as in formula [I] and $R^{2a}$ represents the same meaning as in formula [I] except that it is not hydrogen atom; in formula [IV], X represents halogen such as fluorine, chlorine, bromine or iodine; and in formula [Ia], $R^1$ represents the same meanings as in formula [I], and $R^{2a}$ represents the same meaning as in formula [III])

In formula [III], $R^{2a}$ is preferably $C_1$ - $C_{20}$ alkyl-substituted silyl group, silyl group substituted with $C_1$ - $C_{10}$ alkyl and/or phenyl group, or trityl group, more preferably, $C_1$ - $C_4$ alkyl-substituted silyl group.

Any solvent which does not adversely affect the reaction may be employed as the reaction solvent. Aprotic solvents such as ether, tetrahydrofuran, dioxane, benzene, 1,2-dimethoxyethane and dimethylformamide are preferred.

Preferred examples of the base employed in the reaction include organic bases such as trialkylamines, pyridine, picoline, lutidine, imidazole and potassium t-butoxide; and inorganic bases such as sodium hydride, potassium hydride, potassium hydroxide, sodium hydroxide, sodium carbonate and sodium hydrogen carbonate.

In the above-described reaction equation, the compound of the formula [IV] may be used in an amount 1 - 10 moles, preferably 1 - 5 moles, per 1 mole of the compound of the formula [III], and the base may be used in an amount 1 - 10 moles, preferably 2 - 5 moles, per 1 mole of the compound of the formula [III].

The reaction may be carried out at a temperature from 0°C (cooling in iced water) to the boiling point of the solvent, preferably from 0°C to room temperature. The reaction time may usually be 1 - 72 hours.

Most of the compounds of the formula [III] used as a starting material of the reaction are known compounds. These compounds may be prepared by known methods or by the methods similar to the known methods. That is, the compounds of the formula [III] in which $R^{2a}$ is an unsaturated alkyl-substituted carbonyl, alkyl-substituted benzoyl, $C_1$ - $C_4$ alkyloxycarbonyl, dimethylaminoethyloxycarbonyl, butoxyethoxyacetyl, N-alkylpiperazinylacetyl, N-alkylprolyl, valyl or the like may easily be prepared by reacting the known trifluorothymidine with a corresponding acid halide or acid anhydride in the presence of a base. The compounds of the fomrula [III] in which $R^{2a}$ is a $C_1$ - $C_4$ alkyloxymethyl, benzyl, trityl, propargyl or the like may be prepared by reacting the known trifluorothymidine with a corresponding alkyl halide in the presence of a base. The compounds of the formula [III] in which $R^{2a}$ is a $C_1$ -$C_{20}$ alkyl-substituted silyl group or a silyl group substituted with $C_1$ - $C_{10}$ alkyl and/or phenyl group may be prepared by reacting the known trifluorothymidine with a corresponding silyl halide in the presence of a base. The compounds of the formula [III] in which $R^{2a}$ is a $C_1$ -$C_{30}$ cyclic or chain alkyl-substituted carbamoyl group or diethylaminopropylcarbamoyl group may be prepared by reacting the known trifluorothymidine with a corresponding isocyanate in the presence or absence of a base. Further, the compounds of the fomula [III] in which $R^{2a}$ is an alkyl-substituted phosphoryl may be prepared by reacting the known trifluorothymidine with alkoxyphosphorylhalide in the presence of a base.

The compounds of the formula [I] wherein $R^3$ is $C_1$ -$C_4$ alkyl, benzyl, benzoyl, $C_1$ - $C_4$ alkyloxy-substituted benzoyl, furoyl or $C_1$ - $C_4$ alkyl-substituted carbonyl (i.e., the compounds of the above-described formula [Ib]) may be prepared by reacting the compound [Ia] obtained by the above-described reaction with a corresponding acyl halide or carboxylic acid anhydride in the presence or absence of a solvent and in the presence of a base.

The solvent used here is not restricted and preferred examples thereof include chloroform, dichloromethane, benzene, dimethylformamide, dimethylsulfoxide, ether, THF and dioxane. Preferred examples of the base include inorganic bases such as sodium hydride and organic bases such as trialkylamine and pyridine.

Preferred examples of the acyl halide include benzoyl halide, 4-methoxybenzoyl halide, 2-furoyl halide, acetyl halide and pivaloyl halide. Preferred examples of the carboxylic acid anhydride include acetic anhydride, pivalic anhydride and benzoic anhydride.

The reaction temperature is not restricted and may be from 0°C (i.e., cooled in iced water) to the boiling point of the solvent. The reaction time may be 1 - 72 hours.

The compounds of the formula [I] in which $R^2$ is hydrogen atom (i.e., the compounds of the above-described formula [Ic]) may be prepared by removing the protective group at 5'-position of the compound of the formula [Ia] obtained by the above-described process or of the compound of the formula [I] in which $R^2$ is $C_1$ - $C_{20}$ alkyl-substituted silyl group, silyl group substituted with $C_1$ - $C_{10}$ alkyl and/or phenyl, or trityl group (i.e., the above-described formula [Id]). The deprotection may be carried out in a solvent in the presence of a catalyst. Any solvent which does not adversely affect the reaction may be used. Preferred examples of the solvent include haloalkanes such as dichloromethane, chloroform and dichloroethane; polar solvents such as dimethylformamide, dimethylacetamide, dimethylsulfoxide and acetonitrile; ethers such as diethylether, tetrahydrofuran, dioxane and dimethoxyethane. As the catalyst, various catalysts which are used in this kind of reaction may be employed. Preferred examples of the catalyst which may be employed include fluorides such as tetrabutylammonium fluoride, hydrofluoric acid and cesium fluoride; inorganic acids such as hydrochloric acid and sulfuric acid; organic acids such as toluenesulfonic acid, trifluoromethanesulfonic acid, trifluoroacetic acid and acetic acid; and acidic ion-exchange resins.

The compounds of the formula [I] wherein $R^2$ is not hydrogen atom (i.e., the compounds of the above-described formula [Ie]) may be prepared by reacting the compounds of the formula [Ic] which are produced by the above-mentioned process with a corresponding acyl halide, carboxylic acid anhydride, alkyl halide or the like in the presence or absence of a solvent in the presence of a base.

Any solvent which does not adversely affect the reaction may be used here. Preferred examples of the solvent include chloroform, dichloromethane, benzene, dimethylformamide, dimethylsulfoxide, ether, THF and dioxane.

Preferred examples of the base include inorganic bases such as sodium hydride and organic bases such as trialkylamine and pyridine. Preferred examples of the acyl halide include benzoyl halide, 4-methoxybenzoyl halide, 2-furoyl halide, acetyl halide and pivaloyl halide. Preferred examples of the carboxylic acid anhydride include acetic anhydride, pivalic anhydride and benzoic anhydride. Preferred examples of the alkyl halide include methoxymethyl halide, ethoxymethyl halide, triphenylmethyl halide and benzyl halide.

The reaction temperature is not restricted and may be from 0°C (i.e., cooled in iced water) to the boiling point of the solvent. The reaction time may be 1 - 72 hours.

When the compound of the present invention is used as a pharmaceutical for treating a tumor or for inhibiting a tumor growth, although the dosage of the compound varies depending on the properties of the compound and on the conditions of the patient, the dosage may usually be 50 - 1000 mg/day for an adult in both oral administration and parenteral administration. The above-mentioned dosage may be administered in one time or may be divided into several times.

The compound may be formulated to tablets, granules, powder, suspensions and capsules for oral administration and may be formulated to, for example, suppository for parenteral application. Such pharmaceutical compositions contain effective amount of the compound of the present invention in a pharmaceutically acceptable carrier well-known in the art. For example, for fomulating tablets, crystalline cellulose, soft silicic anhydride or the like may be used as an absorbent, and corn starch, lactose, potassium phosphate, magnesium stearate or the like may be used as a vehicle.

The invention will now be described by way of examples thereof. It should be noted that the examples are presented for the illustration purpose only and should not be interpreted in any restrictive way.

Example 1

5'-O-(t-butyldimethylsilyl)-3'-O-propargyl-α,α,α-trifluorothymidine

(a) 5'-O-(t-butyldimethylsilyl)-α,α,α-trifluorothymidine

A mixture of 197 mg of α,α,α-trifluorothymidine, 110 mg of chloro-t-butyldimethylsilane, 58.9 mg of imidazole and 3.9 ml of DMF was stirred at room temperature for 1 hour. After completion of the reaction,

the reaction mixture was condensed and purified by column chromatography to obtain 5'-O-(t-butyldimethylsilyl)-α,α,α-trifluorothymidine.
Yield: 220 mg

(b) 5'-O-(t-butyldimethylsilyl)-3'-O-propargyl-α,α,α-trifluorothymidine

In 234 ml of THF, 7.8 g of 5'-O-(t-butyldimethylsilyl)-α,α,α-trifluorothymidine was dissolved and 1.75 g of sodium hydride (purity: 60%) was added thereto at room temperature. After 15 minutes, 3.39 g of propargylbromide was added dropwise and the reaction mixture was left to stand for 5 hours at room temperature so as to allow the reaction. After completion of the reaction, the reaction mixture was poured into iced water and extracted with ethyl acetate. The extract was condensed and purified by column chromatography to obtain 5'-O-(t-butyldimethylsilyl)-3'-O-propargyl-α,α,α-trifluorothymidine.
Yield: 7.9 g

## Example 2

### 3'-O-propargyl-α,α,α-trifluorothymidine

In 20 ml of THF, 0.93 g of 3'-O-propargyl-5'-O-(t-butyldimethylsilyl)-α,α,α-trifluorothymidine was dissolved and 2.1 ml of tetra(n-butyl)ammonium fluoride (1M solution in THF) was added to the solution. After allowing the reaction for 11 hours, the reaction product was condensed and the residue was dissolved in ethyl acetate. The resulting solution was washed with iced water, extracted with ethyl acetate, condensed and purified by column chromatography to obtain 3'-O-propargyl-α,α,α-trifluorothymidine.
Yield: 0.85 g

## Example 3

### 3-N:5'-O-dipivaloyl-3'-O-propargyl-α,α,α-trifluorothymidine

In 5.5 ml of dichloromethane, 0.4 g of 3'-O-propargyl-α,α,α-trifluorothymidine was dissolved and 0.35 ml of triethylamine was added thereto. While cooling in iced water, 0.31 ml of pivaloyl chloride was added dropwise to the mixture. After allowing to react at room temperature for 12 hours, saturated aqueous sodium hydrogen carbonate solution was added and the resultant was vigorously stirred. The resultant was extracted with ethyl acetate and then washed with 1N hydrochloric acid and then with saturated aqueous sodium hydrogen carbonate solution. After being condensed, the extract was purified by column chromatography to obtain 3-N:5'-O-dipivaloyl-3'-O-propargyl-α,α,α-trifluorothymidine.
Yield: 0.31 g

## Example 4

### 5'-O-pivaloyl-3'-O-propargyl-α,α,α-trifluorothymidine

In 13 ml of dichloromethane, 430 mg of 3'-O-propargyl-α,α,α-trifluorothymidine, 0.52 g of pyridine and catalytic amount of 4-dimethylaminopyridine were dissolved. While cooling the mixture in iced water, 1.20 g of pivalic anhydride was added dropwise to the mixture. The resulting mixture was stirred for 2 days at room temperature. After adding water to the reaction mixture, the reaction mixture was extracted with dichloromethane and the extract was condensed and purified by column chromatography to obtain 5'-O-pivaloyl-3'-O-propargyl-α,α,α-trifluorothymidine.
Yield: 450 mg

## Example 5

### 5'-O-(t-butyldimethylsilyl)-3'-O-propargyl-3-benzoyl-α,α,α-trifluorothymidine

To a mixture of 5.45 g of 5'-O-(t-butyldimethylsilyl)-3'-O-propargyl-α,α,α-trifluorothymidine, 1.60 g triethylamine and 164 ml of dichloromethane, 1.88 g of benzoyl chloride was added dropwise while cooling the mixture in iced water. After stirring the mixture at room temperature for 3 hours, saturated aqueous sodium hydrogen carbonate solution was added to the mixture and the resultant was extracted with

dichloromethane. The extract was washed with dilute hydrochloric acid and saturated aqueous sodium chloride solution and condensed, followed by purification by column chromatography to obtain 5'-O-(t-butyldimethylsilyl)-3'-O-propargyl-3-benzoyl-α,α,α-trifluorothymidine.
Yield: 4.45 g

Example 6

3-benzoyl-3'-O-propargyl-α,α,α-trifluorothymidine

A mixture of 4.45 g of 5'-O-(t-butyldimethylsilyl)-3'-O-propargyl-3-benzoyl-α,α,α-trifluorothymidine, 45 ml of acetic acid, 22 ml of water and 22 ml of THF was heated at 60°C for 4 hours. After completion of the reaction, the reaction mixture was condensed and toluene was added thereto, followed by condensation of the mixture. The obtained residue was dissolved in dichloromethane and the resulting solution was washed with saturated aqueous sodium hydrogen carbonate solution and saturated aqueous sodium chloride solution. The resultant was condensed and the obtained condensate was recrystallized from ethanol-water to obtain 3'-O-propargyl-3-benzoyl-α,α,α-trifluorothymidine.
Yield: 2.20 g

Example 7

5'-O-isopropyldimethylsilyl-3'-O-propargyl-α,α,α-trifluorothymidine

In 10 ml of pyridine, 0.8 g of 3'-O-propargyl-α,α,α-trifluorothymidine was dissolved and 0.54 ml of isopropyldimethylsilyl chloride was added dropwise to the solution. The resulting mixture was left to stand overnight at room temperature and the resulting mixture was condensed. The obtained residue was dissolved in ethyl acetate and the resultant was washed with water. The resultant was extracted with ethyl acetate and condensed, followed by purification by column chromatography. The obtained product was further purified by recrystallization from ethanol/water (1:1) to obtain 5'-O-isopropyldimethylsilyl-3'-O-propargyl-α,α,α-trifluorothymidine.
Yield: 565 mg

ALTERNATIVE METHOD

(a) 5'-O-isopropyldimethylsilyl-α,α,α-trifluorothymidine

To a solution containing 1.0 g of α,α,α-trifluorothymidine, 1.3 g of imidazole and 10 ml of DMF, 0.58 ml of isopropyldimethylsilyl chloride was added dropwise. The resulting mixture was left to stand overnight at room temperature and then the reaction mixture was condensed. The obtained residue was dissolved in ethyl acetate and washed with water. The resultant was extracted with ethyl acetate and condensed, followed by recrystallization from ethanol/water (1:1) to obtain 5'-O-isopropyldimethylsilyl-α,α,α-trifluorothymidine.
Yield: 640 mg

b) 5'-O-isopropyldimethylsilyl-3'-O-propargyl-α,α,α-trifluorothymidine

In 5 ml of THF, 0.64 g of 5'-O-isopropyldimethylsilyl-α,α,α-trifluorothymidine and 103 mg of sodium hydride were dissolved and the resulting mixture was stirred at room temperature for 15 minutes. To this mixture, 0.167 ml of propargyl bromide was added dropwise and the resulting mixture was left to stand overnight at room temperature. Then the reaction mixture was poured into iced water and extracted with ethyl acetate. The organic layer was washed with saturated aqueous ammonium chloride solution and dried over anhydrous sodium sulfate. The obtained crude oily product was purified to crystals in the same manner as described above to obtain 5'-O-isopropyldimethylsilyl-3'-O-propargyl-α,α,α-trifluorothymidine.
Yield: 586 mg

Example 8

5'-O,N,N-octadecylmethylcarbamoyl-3'-O-propargyl-α,α,α-trifluorothymidine

In 30 ml of THF, 1.41 g of 3'-O-propargyl-α,α,α-trifluorothymidine and 0.89 g of 1,1'-carbonyldiimidazole were added and the resulting mixture was left to stand overnight at room temperature. Then 2.4 g of octadecylmethylamine was added and the resulting mixture was left to stand overnight at room temperature. Thereafter, the reaction mixture was condensed and the obtained residue was dissolved in ethyl acetate. The resultant was washed with water and extracted with ethyl acetate. The extract was condensed and purified by column chloromatography. The obtained product was further purified by recrystallization from ether/ethanol to obtain 2-deoxy-5'-O-N,N-methyloctadecylcarbamoyl-3'-O-propargyl-α,α,α-trifluorothymidine.
Yield: 1.96 g

Example 9

5'-O-palmitoyl-3'-O-propargyl-α,α,α-trifluorothymidine

To a solution containing 1.5 g of 3'-O-propargyl-α,α,α-trifluorothymidine in 20 ml of pyridine, 1.48 g of palmitoyl chloride was added dropwise while cooling the solution in iced water. The resulting mixture was left to stand overnight at room temperature. Then iced water was added to the reaction mixture and the resultant was extracted with ethyl acetate. The resultant was washed with water and condensed, followed by purification by column chromatography to obtain 5'-O-palmitoyl-3'-O-propargyl-α,α,α-trifluorothymidine.
Yield: 1.79 g

Example 10

3'-O-propargyl-5'-O-lignoceroyl-α,α,α-trifluorothymidine

To a suspension containing 0.54 g of 3'-O-propargyl-α,α,α-trifluorothymidine, 660 mg of lignoceric acid and a catalytic amount of N-dimethylaminopyridine in 15 ml of dichloromethane, 370 mg of N,N'-dicyclohexylcarbodiimide was added at room temperature. The resulting mixture was left to stand overnight at room temperature and the insoluble materials were removed by filtration. The obtained filtrate was condensed and the resulting residue was recrystallized from methanol/water (4:1) to obtain 3'-O-propargyl-5'-O-lignoceroyl-α,α,α-trifluorothymidine.
Yield: 1.10 g

Example 11

5'-O-{2-(4-nonylpiperazin-1-yl)acetyl}-3'-O-propargyl-α,α,α-trifluorothymidine dihydrochloride

(a) 1-nonanoylpiperazine

To a solution containing 5.34 g of nonanoyl chloride and 5.33 g of 2,4-dichlorophenol in 160 ml of 1,2-dichloroethane, 3.61 g of triethylamine was added dropwise while cooling the solution in iced water. After stirring the mixture for 2 hours, a solution containing 12.8 g of piperazine in 50 ml of 1,2-dichloroethane was added dropwise thereto. After stirring the resulting mixture for 2 hours, insoluble materials were removed by filtration and the obtained filtrate was sequentially washed with water, 10% aqueous sodium hydroxide solution, water and saturated aqueous sodium chloride solution. After drying the resultant over potassium carbonate, the resultant was condensed to obtain 1-nonanoylpiperazine.
Yield: 6.9 g

(b) 1-nonylpiperazine

To a suspension containing 1.76 g of lithiumaluminum hydride in 50 ml of THF, a solution containing 7.0 g of 1-nonanoylpiperazine in 90 ml of THF was added dropwise while cooling. After stirring the resulting mixture at room temperature for 2 hours, excess reagents were quenched by water-containing ether and the resultant was filtered through Celite. By condensing the filtrate, 1-nonylpiperazine was obtained as pale yellow oil.
Yield: 5.28 g

(c) ethyl 2-(4-nonylpiperazin-1-yl) acetate

A solution containing 3.0 g of 1-nonylpiperazine, 2.48 g of ethyl α-bromoacetate and 1.57 g of triethylamine in 90 ml of THF was stirred at room temperature for 1.5 hours. After completion of the reaction, the insoluble materials were removed by filtration and the obtained filtrate was condensed. The obtained residue was purified by column chromatography to obtain 2-(4-nonylpiperazin-1-yl)ethyl acetate.
Yield: 2.90 g

(d) 2-(4-nonylpiperazin-1-yl)acetic acid

To a solution containing 2.90 g of ethyl 2-(4-nonylpiperazine-1-yl) acetate in 87 ml of ethanol, 87 ml of aqueous solution containing 2.73 g of potassium hydroxide was added and the resulting mixture was stirred at room temperature for 1 hour. After completion of the reaction, ethanol was evaporated off and the resultant was neutralized with hydrochloric acid. The resultant was extracted with butanol. After evaporating the solvent, methanol was added and the generated insoluble materials were removed by filtration. The obtained filtrate was condensed to obtain 2-(4-nonylpiperazin-1-yl)acetic acid.
Yield: 2.75 g

(c) 5'-O-{2-(4-nonylpiperazin-1-yl)acetyl}-3'-O-propargyl-α,α,α-trifluorothymidine dihydrochloride

To a solution containing 1.03 g of 3'-O-propargyl-α,α,α-trifluorothymidine, 1.25 g of 2-(4-nonylpiperazine-1-yl)acetic acid and 935 mg of triethylamine in 31 ml of 1,2-dichloroethane, 1.18 g of bis(2-oxo-3-oxazolidinyl)phosphinyl chloride was added. The resulting mixture was stirred at room temperature for 3 hours and then heated to reflux for 6 hours. Then the reaction mixture was condensed and water was added thereto. The resultant was extracted with ethyl acetate and the extract was washed with aqueous sodium hydrogen carbonate solution, followed by drying over magnesium sulfate. After evaporating the solvent, the resultant was purified by column chromatography. The obtained product in ethyl acetate was treated with 4N hydrochloric acid/dioxane solution to obtain 5'-O-{2-(4-nonylpiperazin-1-yl)acetyl}-3'-O-propargyl-α,α,α-trifluorothymidine dihydrochloride.
Yield: 0.75 g

Example 12

5'-O-{2-(2-butoxyethoxy)acetyl}-3'-O-propargyl-α,α,α-trifluorothymidine

(a) t-butyl 2-(2-butoxyethoxy) acetate

To a mixture of 1.18 g of 2-butoxyethanol, 20 ml of 50% aqueous sodium hydroxide solution, 19.5 g of t-butyl α-bromoacetate, 3.4 g of tetrabutylammonium hydrogensulfate and 3.2 ml of dichloromethane, 20 ml of 50% aqueous sodium hydroxide solution was added and the mixture was stirred at room temperature for 24 hours. After completion of the reaction, water was added to the reaction mixture and the reaction mixture was extracted with ethyl acetate. The resultant was condensed and purified by column chromatography to obtain t-butyl 2-butoxyethoxy) acetate.
Yield: 2.3 g

(b) 2-(2-butoxyethoxy)acetic acid

To a solution containing 1.42 g of t-butyl 2-(2-butoxyethoxy) acetate in 43 ml of dichloromethane, 1.4 ml of trifluoroacetic acid was added while cooling the solution in iced water and the resulting mixture was stirred at room temperature for 5 hours. After completion of the reaction, the reaction mixture was condensed and the obtained residue was purified by column chromatography to obtain 2-(2-butoxyethoxy)-acetic acid.
Yield: 1.0 g

(c) 5'-O-{2-(2-butoxyethoxy)acetyl}-3'-O-propargyl-α,α,α-trifluorothymidine

To a solution containing 1.07 g of 3'-O-propargyl-α,α,α-trifluorothymidine, 848 mg of 2-(2-butoxyethoxy)-acetic acid and 974 mg of triethylamine in 32 ml of THF, 1.23 g of bis(2-oxo-3-oxazolidinyl)phosphinyl chloride was added. The resulting solution was stirred for 3 hours at room temperature and then heated to reflux for another 3 hours. Then the reaction mixture was condensed and water was added thereto. The

resultant was extracted with ethyl acetate and the extract was washed with aqueous sodium hydrogen carbonate solution, followed by drying over magnesium sulfate. After evaporating the solvent, the residue was purified by column chromatography to obtain 5'-O-{2-(2-butoxyethoxy)acetyl}-3'-O-propargyl-α,α,α-trifluorothymidine.
Yield: 0.81 g

Example 13

5'-O-(triethylsilyl)-3'-O-propargyl-α,α,α-trifluorothymidine

To a solution containing 1.03 g of 3'-O-propargyl-α,α,α-trifluorothymidine in 21 ml of pyridine, 511 mg of chlorotriethylsilane was added dropwise while cooling the solution in iced water, and the resulting mixture was stirred at room temperature for 3 hours. After completion of the reaction, the reaction mixture was condensed and water was added thereto. The resultant was extracted with ethyl acetate and the extract was washed with 10% hydrochloric acid and then with saturated aqueous sodium chloride solution. The resultant was dried over magnesium sulfate and condensed. The obtained residue was purified by column chromatography to obtain 5'-O-(triethylsilyl)-3'-O-propargyl-α,α,α-trifluorothymidine.
Yield: 1.0 g

Example 14

5'-O-(N-dodecyl-L-prolyl)-3'-O-propargyl-α,α,α-trifluorothymidine hydrochloride

(a) N-dodecyl-L-proline

To a solution containing 1.66 g of (S)-L-proline in 50 ml of ethanol, 3.19 g of bromododecane and 2.19 g of potassium carbonate were added and the resulting mixture was stirred at 80°C for 2 hours. After completion of the reaction, the insoluble materials were removed by filtration and the filtrate was condensed. The obtained residue was purified by flash column chromatography to obtain N-dodecyl-L-proline.
Yield: 2.32 g

(b) 5'-O-(N-dodecyl-L-prolyl)-3'-O-propargyl-α,α,α-trifluorothymidine hydrochloride

A solution containing 1.15 g of 3'-O-propargyl-α,α,α-trifluorothymidine, 1.06 g of N,N'-dicyclohexylcarbodiimide, 697 mg of 1-hydroxybenz triazole and 1.47 g of N-dodecyl-L-proline in 35 ml of THF was stirred at room temperature for 24 hours. After completion of the reaction, the insoluble materials were removed by filtration, and the filtrate was condensed. The obtained residue was dissolved in ethyl acetate and the resulting solution was washed with aqueous sodium hydrogen carbonate solution and saturated aqueous sodium chloride solution. The resultant was dried over magnesium sulfate and condensed. The obtained residue was purified by column chromatography. The obtained product was dissolved in ethyl acetate and treated with 4N hydrochoric acid/dioxane to obtain 5'-O-(N-dodecyl-L-prolyl)-3'-O-propargyl-α,α,α-trifluorothymidine hydrochloride.
Yield: 1.53 g

Example 15

5'-O-(D-valyl)-3'-O-propargyl-α,α,α-trifluorothymidine hydrochloride

(a) 5'-O-(N-tert-butoxycarbonyl-D-valyl)-3'-O-propargyl-α,α,α-trifluorothymidine

A mixture of 930 mg of 3'-O-propargyl-α,α,α-trifluorothymidine, 665 mg of N-tert-butoxycarbonyl-D-valine, 631 mg of N,N'-dicyclohexylcarbodiimide, catalytic amount of 4-dimethylaminopyridine, 28 ml of dichloromethane and 25 ml of THF was stirred at room temperature for 1.5 hours. After completion of the reaction, the reaction mixture was condensed and the residue was purified by column chromatography to obtain 5'-O-(N-tert-butoxycarbonyl-D-valyl)-3'-O-propargyl-α,α,α-trifluorothymidine.
Yield: 1.32 g

(b) 5'-O-(D-valyl)-3'-O-propargyl-α,α,α-trifluorothymidine hydrochloride

A solution containing 1.32 g of 5'-O-(N-tert-butoxycarbonyl-D-valyl)-3'-O-propargyl-α,α,α-trifluorothymidine in 4 ml of 4N hydrochloric acid/dioxane was stirred at 80°C for 30 minutes. After completion of the reaction, the reaction mixture was condensed and the obtained oily product was crystallized with ether to obtain 5'-O-(D-valyl)-3'-O-propargyl-α,α,α-trifluorothymidine hydrochloride.
Yield: 0.92 g

Example 16

3,5'-bis(methoxymethyl)-3'-O-propargyl-α,α,α-trifluorothymidine

To a solution containing 1.04 g of 3'-O-propargyl-α,α,α-trifluorothymidine and 3.25 ml of ethyldiisopropylamine in 31 ml of dichloromethane, 1.2 ml of methoxymethylbromide was added dropwise while cooling the solution in iced water, and the resulting solution was stirred at room temperature for 24 hours. Water was added to the reaction mixture and the resulting mixture was extracted with ethyl acetate. The resultant was washed with saturated aqueous sodium chloride solution and dried over magnesium sulfate. The resultant was condensed and the obtained residue was purified by column chromatography to obtain 3,5'-bis(methoxymethyl)-3'-O-propargyl-α,α,α-trifluorothymidine.
Yield: 630 mg

Example 17

5'-O-methoxymethyl-3'-O-propargyl-α,α,α-trifluorothymidine

To a suspension containing sodium hydride (150 mg as 60% oil) in 12 ml THF, 568 mg of 3'-O-propargyl-α,α,α-trifluorothymidine in 5 ml of THF was added dropwise at room temperature Fifteen minutes later, 1.2 ml of butyllithium (1.4 M solution in n-hexane) was added dropwise at -78°C. Five minutes later, 144 mg of chloromethylmethylether was added dropwise. The resulting mixture was stirred at room temperature for 1 hour and iced water was added to the reaction mixture. The resulting mixture was condensed and extracted with ethyl acetate. The extract was washed with saturated aqueous sodium chloride solution and dried over magnesium sulfate. The resultant was condensed and the residue was purified by column chromatography to obtain 5'-O-methoxymethyl-3'-O-propargyl-α,α,α-trifluorothymidine.
Yield: 227 mg

Example 18

5'-O-dodecyloxyhydroxyphosphoryl-3'-O-propargyl-α,α,α-trifluorothymidine

To 18 ml of pyridine, 1.13 ml of methyldichlorophosphate was added dropwise at room temperature and the mixture was stirred for 15 minutes. To this mixture, 3.6 ml of dodecanol was added dropwise and the resulting mixture was stirred at room temperature for 1 hour. Then 0.3 g of 3'-O-propargyl-α,α,α-trifluorothymidine was added and the resulting mixture was stirred at room temperature for 2 days. After completion of the reaction, water was added to the reaction mixture and the resultant was extracted with ethyl acetate. The resultant was condensed and the obtained residue was purified by column chromatography to obtain 5'-O-dodecyloxyhydroxyphosphoryl-3'-O-propargyl-α,α,α-trifluorothymidine.
Yield: 90 mg

Example 19

5'-O-(2-oxo-1,3-dioxaphospholan-2-yl)-3'-O-propargyl-α,α,α-trifluorothymidine

In 16 ml of dichloromethane, 0.5 g of 3'-O-propargyl-α,α,α-trifluorothymidine was dissolved. To this solution, 0.36 ml of triethylamine and 0.32 g of 2-chloro-2-oxo-1,3-dioxaphospholane were added. The resulting mixture was left to stand overnight at room temperature and then condensed. The residue was purified by column chromatography to obtain 5'-O-(2-oxo-1,3-dioxaphospholan-2-yl)-3'-O-propargyl-α,α,α-trifluorothymidine.
Yield: 120 mg

Example 20

3',5'-di-O-propargyl-α,α,α-trifluorothymidine

In 15 ml of THF, 0.9 g of sodium hydride was suspended and 0.75 ml of propargylalcohol was added dropwise to the suspension. This mixture was added to a solution cooled in iced water, which contained 1.5 ml of trichloroacetonitrile in 15 ml of ether and the resultant was left to stand for 2.5 hours. Then 50 ml of pentane containing a small amount of methanol was added to the reaction mixture and the generated amorphous powder was removed by filtration. The obtained filtrate was condensed and 15 ml of dichloromethane was added thereto. In this mixture, 0.4 g of 3'-O-propargyl-α,α,α-trifluorothymidine was dissolved and 6 drops of trimethylsilyltriflate were addded. The reaction mixture was left to stand at room temperature for 4 hours. The reaction mixture was then washed with saturated sodium hydrogen carbonate solution and dried over sodium sulfate. The resultant was condensed and purified by column chromatography to obtain 3',5'-di-O-propargyl-α,α,α-trifluorothymidine.
Yield: 142 mg

Example 21

5'-O-butoxycarbonyl-3'-O-propargyl-α,α,α-trifluorothymidine

To a solution containing 519 mg of 3'-O-propargyl-α,α,α-trifluorothymidine in 16 ml of pyridine, 636 mg of butyl chloroformate was added dropwise while cooling the solution in iced water. The resulting mixture was stirred at room temperature for 2 days and then condensed. Water was added to the reaction mixture and the resulting mixture was extracted with ethyl acetate. The resultant was washed with saturated aqueous sodium chloride solution and dried over magnesium sulfate. The resultant was condensed and the obtained residue was purified by column chromatography to obtain 5'-O-butoxycarbonyl-3'-O-propargyl-α,α,α-trifluorothymidine.
Yield: 380 mg

Example 22

5'-O-(N-cyclohexylcarbamoyl)-3'-O-propargyl-α,α,α-trifluorothymidine

A solution containing 1.64 g of 3'-O-propargyl-α,α,α-trifluorothymidine and 1.03 g of 1,1'-carbonyldiimidazole in 49 ml of THF was stirred at room temperature for 6 hours. To this solution, 1.95 g of cyclohexylamine was added dropwise and the resulting mixture was heated to reflux under stirring for 7 hours. After completion of the reaction, the reaction mixture was condensed and 10% hydrochloric acid was added to the obtained residue. The resultant was then extracted with ethyl acetate and the extract was washed with saturated aqueous sodium chloride solution, followed by drying over anhydrous magnesium sulfate. The resultant was condensed and the obtained residue was purified by column chromatography, followed by further purification by recrystallization from ethanol/water to obtain 5'-O-(N-cyclohexylcarbamoyl)-3'-O-propargyl-α,α,α-trifluorothymidine.
Yield: 1.24 g

Example 23

3-ethyl-5'-O-(N-cyclohexylcarbamoyl)-3'-O-propargyl-α,α,α-trifluorothymidine

To a solution containing 82.1 mg of cesium fluoride in 3.2 ml of DMF, 108 mg of 3'-O-propargyl-α,α,α-trifluorothymidine and 40.3 mg of ethyl iodide were added, and the resulting mixture was stirred at room temperature for 1 hour. After completion of the reaction, the reaction mixture was condensed and extracted with ethyl acetate. The extract was washed with saturated aqueous sodium chloride solution and dried over magnesium sulfate. The resultant was condensed and the obtained residue was purified by column chromatography to obtain 3-ethyl-5'-O-(N-cyclohexylcarbamoyl)-3'-O-propargyl-α,α,α-trifluorothymidine.
Yield: 100 mg

The identification date of the products prepared in Examples 1 - 23 are summarized in Table 1.

Examples 24 - 47

Various compounds were prepared in the similar manner as in Examples 1 - 23. The prepared

compounds as well as their identification data are shown in Table 1.

Table 1

| Ex. No. | $R^1$ : | $R^3$ : | $R^2$ : | N M R (solvent used in measurement) ; δ ppm | I R $cm^{-1}$ | Melting Point (℃) |
|---|---|---|---|---|---|---|
| 1 | H | H | 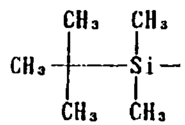 | ($CDCl_3$) 0.092 (3H, s), 0.097 (3H, s), 0.897 (9H, s), 1.96-2.02 (1H, m), 2.47-2.48 (1H, m), 2.62-2.67 (1H, m), 3.81-3.96 (2H, m), 4.16-4.37 (4H, m), 6.17 (1H, dd), 8.25 (1H, s), 8.62 (1H, bs) | (neat) 3298, 2959, 1706, 1650, 1473 | 87-88 |
| 2 | H | H | H | ($CDCl_3$) 2.18-2.28 (1H, m), 2.48-2.64 (2H, m), 3.77-3.94 (2H, m), 4.08-4.42 (4H, m), 6.23-6.27 (1H, m), 8.72 (1H, s) | (KBr) 3420, 2920, 2120, 1720, 1680 | 178-179 |
| 3 | H | pivaloyl | pivaloyl | ($CDCl_3$) 1.21 (9H, s), 1.33 (9H, m), 2.04 (1H, m), 2.49 (2H, m), 2.72 (1H, m), 4.15-4.41 (6H, m), 6.10 (1H, br), 8.02 (1H, s) | (neat) 2973, 1781, 1685 | |
| 4 | H | H | pivaloyl | ($CDCl_3$) 1.21 (9H, s), 1.98 (1H, ddd), 2.49 (1H, t), 2.72 (1H, ddd), 4.20 (1H, dd), 4.24 (1H, dd), 4.23-4.32 (2H, m), 4.33-4.42 (2H, m), 6.13 (1H, dd), 8.00 (1H, s), 8.79 (1H, br) | (neat) 3265, 3089, 2977, 2119, 1738, 1698 | |
| 5 | H | benzoyl | 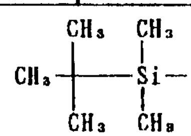 | ($CDCl_3$) 0.119 (3H, s), 0.123 (3H, s), 0.927 (9H, s), 2.06 (1H, ddd), 2.46 (1H, t), 2.67 (1H, ddd), 3.85 (1H, dd), 3.97 (1H, dd), 4.17 (1H, dd), 4.22 (1H, dd), 4.28 (1H, bd), 4.37 (1H, bd), 6.18 (1H, dd), 7.52 (2H, t), 7.68 (1H, bd), 7.93 (1H, dd), 8.35 (1H, s) | (neat) 3289, 3072, 2119, 1756, 1716, 1683 | |

Table 1 (continued)

| Ex. No. | R1 : | R3 : | NMR (solvent used in measurement) ; δ ppm | IR $cm^{-1}$ | Melting Point (℃) |
|---|---|---|---|---|---|
| | R2 : | | | | |
| 6 | H | benzoyl | ($CDCl_3$) 2.30 (1H, dt), 2.49 (1H, t), 2.60 (1H, ddd), 3.92 (1H, dd), 4.06 (1H, dd), 4.19 (1H, dd), 4.23 (1H, dd), 4.25 (1H, dt), 4.46 (1H, dt), 6.23 (1H, t), 7.52 (2H, t), 7.68 (1H, dt), 7.93 (2H, bd), 8.73 (1H, s) | (KBr) 3286, 2119, 1754, 1713, 1678 | 129-132 |
| | H | | | | |
| 7 | H | H | ($CDCl_3$) 0.96 (7H, m), 2.03 (1H, m), 2.47 (1H, m), 2.66 (1H, m), 3.77 (1H, dd), 3.94 (1H, dd), 4.16 (1H, dd), 4.24 (1H, dd), 4.27 (1H, m), 4.35 (1H, d), 6.23 (1H, dd), 8.36 (1H, s), 8.69 (1H, br) | (KBr) 3289, 2959, 1706, 1475, 1338, 1286, 1068, 926 | 62-63 |
| | 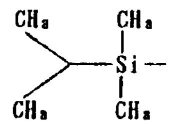 | | | | |
| 8 | H | H | ($CDCl_3$) 0.88 (3H, m), 1.28 (30H, m), 1.51 (2H, m), 1.97 (1H, m), 2.72 (2H, m), 2.47 (1H, m), 2.84 (3H, s), 3.20 (4H, ), 4.17-4.39 (6H, m), 6.16 (1H, t), 8.05 (1H, s), 8.09 (1H, br) | (KBr) 3253, 2921, 1716, 1467, 1164, 1099, 765, 722 | 78-79 |
| | 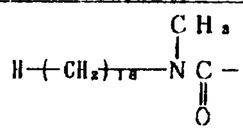 | | | | |
| 9 | H | H | ($CDCl_3$) 0.88 (3H, m), 1.26 (24H, m), 1.58-1.64 (2H, m), 2.05 (1H, m), 2.32 (2H, m), 2.49 (1H, m), 2.68 (1H, m), 4.10-4.44 (6H, m), 6.19 (1H, t), 8.10 (1H, s), 8.88 (1H, br) | (neat) 3214, 2050 1700, 1503, 1324, 1107, 1045, 994 | |
| | 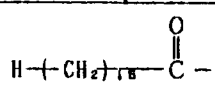 | | | | |
| 10 | H | H | ($CDCl_3$) 0.88 (3H, m), 1.28 (40H, m), 1.58-1.62 (2H, m), 1.78 (2H, m), 2.06 (1H, m), 2.33 (1H, m), 2.54 (1H, m), 2.70 (1H, m), 4.18-4.48 (6H, m), 6.21 (1H, t, ), 8.09 (1H, s) | (KBr) 3468, 3089, 2119, 1705, 1656, 1413, 1316, 956 | 61-63 |
| | 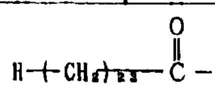 | | | | |

Table 1 (continued)

| Ex. No. | R1 : | R3 : | NMR (solvent used in measurement) ; δ ppm | IR cm-1 | Melting Point (℃) |
|---|---|---|---|---|---|
| | R2 : | | | | |
| 11 | H | H | (DMSO-d6) 0.86 (3H, t), 1.26 (14H, bs), 1.68 (2H, bs), 2.37 (1H, m), 2.45 (1H, m), 2.51 (1H, t), 2.95-3.30 (6H, m), 3.50 (2H, m), 3.9-4.5 (8H, m), 6.03 (1H, t), 8.13 (1H, s) | (KBr) 3422, 2928, 2857, 2114, 1701, 1471, 1280, 1214 | 124-130 |
| | 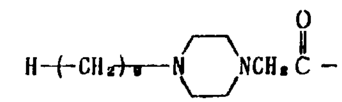 | | | | |
| 12 | H | H | (CDCl$_3$) 0.91 (6H, q), 1.35 (2H, m), 1.55 (2H, m), 2.20 (1H, ddd), 2.51 (1H, t), 2.60 (1H, m), 3.40 (2H, m), 3.55 (2H, m), 3.70 (2H, m), 4.20-4.70 (8H, m), 6.22 (1H, dd), 8.09 (1H, s), 8.66 (1H, bs) | (neat) 3266, 3085, 2936, 2874, 2119, 1705, 1468, 1280 | |
| | 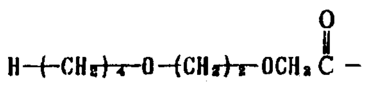 | | | | |
| 13 | H | H | (CDCl$_3$) 0.64 (6H, q), 0.96 (9H, t), 2.03 (1H, ddd), 2.48 (1H, t), 2.62 (1H, ddd), 3.81 (1H, dd), 3.95 (1H, dd) 4.19 (1H, dd), 4.23 (1H, dd), 4.26 (1H, bd), 4.37 (1H, m), 6.22 (1H, dd), 8.36 (1H, s), 8.69 (1H, bs) | (KBr) 3192, 3067, 2963, 2881, 1706, 1474, 1287, 1137 | |
| | $(CH_3CH_2)_3Si-$ | | | | |
| 14 | H | H | (CDCl$_3$) 0.883 (3H, t), 1.25 (20H, bs), 1.70 (4H, m), 2.10 (2H, m), 2.35 (2H, m), 2.63 (1/2H, t), 2.64 (1/2H, t), 2.90 (1H, m), 3.20 (1H, m), 3.80 (1H, m), 4.20-4.30 (3H, m), 4.40 (1H, m), 4.50-4.70 (2H, m), 6.01 (1/2H, t), 7.84 (1/2H, s), 7.99 (1/2H, s), 8.63 (1/2H, bs), 8.87 (1/2H, bs) | (KBr) 2927, 2856, 2113, 1702, 1468, 1411, 1375, 1278, | 86-93 |
| | 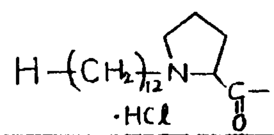 | | | | |
| 15 | H | H | (CDCl$_3$) 1.07 (3H, d), 1.08 (3H, d), 2.38 (2H, m), 2.55 (1H, m), 2.66 (1H, t), 4.25 (3H, m), 4.35 (1H, dd), 4.50 (1H, m), 4.67 (1H, dd), 6.07 (1H, t), 8.00 (1H, s), 11.7 (1H, br) | (KBr) 3430, 2971, 2939, 2117, 1702, 1472, 1279, 1134 | 91-100 |
| | 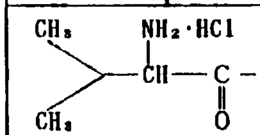 | | | | |

Table 1 (continued)

| Ex. No. | $R^1$ : | $R^3$ : | $R^2$ : | N M R (solvent used in measurement) ; δ ppm | I R $cm^{-1}$ | Melting Point (℃) |
|---|---|---|---|---|---|---|
| 16 | H | methoxymethyl | methoxymethyl | ($CDCl_3$) 2.21 (1H, dt), 2.48 (1H, t), 2.60 (1H, ddd), 3.38 (3H, s), 3.45 (3H, s), 3.80 (1H, dd), 4.19 (1H, dd), 4.24 (1H, dd), 4.28 (1H, m), 4.38 (1H, m), 4.69 (1H, d), 4.68 (1H, d), 5.37 (2H, s), 6.29 (1H, t), 8.53 (1H, s) | (neat) 3269, 3079, 2945, 2832, 2118, 1729, 1683, 1469 | |
| 17 | H | H | methoxymethyl | ($CDCl_3$) 2.20 (1H, dt), 2.49 (1H, t), 2.58 (1H, ddd), 3.80 (1H, dd), 3.86 (1H, dd), 4.20 (1H, dd), 4.23 (1H, dd), 4.28 (1H, dt), 4.39 (1H, ddd), 4.68 (1H, dd), 4.69 (1H, d), 6.28 (1H, t), 8.45 (1H, bs), 8.52 (1H, s) | (neat) 3270, 3076, 2945, 2119, 1732, 1690 | |
| 18 | H | H | 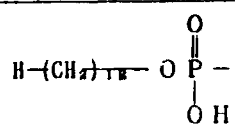 | ($CDCl_3$) 0.92 (3H, t), 1.17 (20H, m), 1.47 (2H, m), 2.35 (1H, m), 2.59 (2H, m), 3.71 (2H, bs), 4.12 (1H, m), 4.34 (2H, m), 5.46 (1H, m), 6.32 (1H, t), 8.30 (1H, s) | | |
| 19 | H | H | 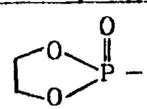 | ($CDCl_3$) 2.14 (1H, m), 2.49 (1H, m), 2.58 (1H, m), 3.86-4.21 (4H, m), 4.58 (4H, m), 6.22 (1H, m), 8.17 (1H, s) | (KBr) 3447, 2287, 1621, 1600, 1524, 1310, 1155, 1021, | |
| 20 | H | H | $HC\equiv CCH_2-$ | ($CDCl_3$) 2.04 (2H, m), 2.57 (1H, m), 3.74 (1H, dd), 3.91 (1H, dd), 4.09 (1H, m), 4.19 (4H, m), 4.23 (1H, m), 6.25 (1H, t), 8.59 (1H, s), 8.95 (1H, bs) | (KBr) 3473, 2285, 1626, 1574, 1301, 1278, 1089, 676 | |

Table 1 (continued)

| Ex. No. | R¹ : / R³ : / R² : | NMR (solvent used in measurement) ; δ ppm | IR $cm^{-1}$ | Melting Point (℃) |
|---|---|---|---|---|
| 21 | R¹: H; R³: H<br>R²: 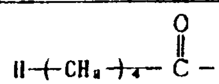 | ($CDCl_3$) 0.94(1H,t), 1.39(1H,sixtet), 1.65(1H,quintet), 2.13(1H,ddd), 2.50(1H,t), 2.63(1H,ddd), 4.16-4.30(4H,m), 4.37(2H,bs), 4.40(2H,s), 6.25(1H,dd), 8.15(1H,s), 8.86(1H,bs) | (neat) 3270, 3087, 2364, 2876, 2119, 1706, 1468, 1408 | |
| 22 | R¹: H; R³: H<br>R²: 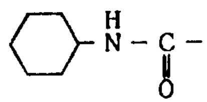 | ($CDCl_3$) 1.12(3H,m), 1.35(2H,m), 1.6(1H,m), 1.7(2H,m), 1.9(2H,m), 2.08(1H,dt), 2.49(1H,bd), 2.65(1H,ddd), 3.47(1H,m), 4.21(1H,bd), 4.23(1H,dd), 4.3(2H,m), 4.43(1H,dd), 4.58(1H,d), 6.18(1H,t), 8.2(1H,s), 8.71(1H,bs) | (KBr) 3367, 2936, 2370, 1707, 1535, 1473, 1283, | 135-136 |
| 23 | R¹: H; R³: ethyl<br>R²: 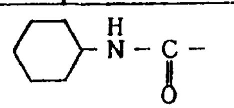 | ($CDCl_3$) 1.14(3H,m), 1.24(3H,t), 1.35(2H,m), 1.61(1H,m), 1.70(2H,m), 1.90(2H,m), 2.08(1H,dt), 2.49(1H,t), 2.66(1H,ddd), 3.47(1H,m), 4.01(2H,m), 4.21(1H,dd), 4.22(1H,dd), 4.30(2H,m), 4.42(1H,dd), 4.57(1H,d), 6.20(1H,t), 8.16(1H,s) | | |
| 24 | R¹: H; R³: acetyl<br>R²: acetyl | ($CDCl_3$) 2.08(S,3H), 2.10-2.28(m,2H), 2.61(m,3H), 2.68(m,1H), 4.11-4.33(m,6H), 6.17(t,1H), 8.11(s,1H) | (neat) 2961, 1798, 1723, 1683 | |
| 25 | R¹: H; R³: H<br>R²: acetyl | ($CDCl_3$) 2.11(3H,s), 2.10(1H,dt), 2.50(1H,t), 2.67(1H,ddd), 4.20(1H,dd), 4.24(1H,dd), 4.30(1H,dt), 4.31(1H,dd), 4.36(1H,q), 4.40(1H,dd), 6.20(1H,t), 8.10(1H,s), 8.90(1H,bs) | (neat) 3272, 3085, 2954, 2119, 1705 | |

Table 1 (continued)

| Ex. No. | R¹ : | R³ : | NMR (solvent used in measurement) ; δ ppm | IR cm⁻¹ | Melting Point (℃) |
|---|---|---|---|---|---|
| | R² : | | | | |
| 26 | H | H | ($CDCl_3$) 2.04 (1H, m), 2.14 (1H, m), 2.71 (1H, m), 3.82 (2H, dd), 3.93 (1H, dd), 4.12 (2H, t), 4.19 (1H, m), 4.33 (1H, d), 6.14 (1H, dd), 7.27 (2H, d), 7.67 (4H, d), 8.18 (1H, s), 9.42 (1H, br) | (neat) 3220, 1709, 1479, 1329, 1288, 1126, 1013, 882 | |
| | 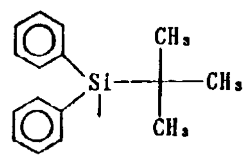 | | | | |
| 27 | H | H | ($CDCl_3$) 1.11-1.36 (6H, m), 1.42-1.84 (5H, m), 2.06 (1H, m), 2.22 (2H, m), 2.61 (1H, m), 2.71 (1H, m), 4.17-4.45 (6H, m), 6.20 (1H, t), 8.10 (1H, s), 9.47 (1H, bs) | (neat) 3267, 2956, 1695, 1470, 1391, 1199, 1042, 926 | |
| | 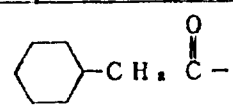 | | | | |
| 28 | H | H | ($CDCl_3$) 0.88 (3H, t), 1.28 (18H, m), 2.32 (1H, m), 2.49 (1H, m), 1.69 (1H, m), 4.16-4.44 (1H, m), 6.19 (1H, t), 8.10 (1H, s), 8.94 (1H, br) | (neat) 3254, 1667, 1533, 1153, 1036, 912, 672 | |
| | 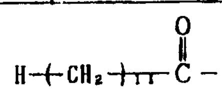 | | | | |
| 29 | H | H | ($CDCl_3$) 0.86 (3H, m), 1.25 (28H, m), 1.58-1.68 (2H, m), 2.06 (1H, m), 2.30 (2H, m), 2.49 (1H, m), 2.67 (1H, m), 4.10-4.44 (6H, m), 6.19 (1H, t), 8.10 (1H, s), 8.88 (1H, br) | (KBr) 3269, 2920, 2850, 1690, 1467, 1284, 1198, 1019, | 68-69 |
| | 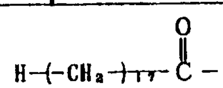 | | | | |
| 30 | H | H | ($CDCl_3$) 0.88 (3H, m), 1.27 (8H, m), 1.61 (2H, m), 1.77 (2H, br), 2.06 (1H, m), 2.33 (2H, m), 2.60 (1H, s), 2.70 (1H, m), 4.16-4.44 (6H, m), 6.20 (1H, t), 8.09 (1H, s), 9.23 (1H, br) | (neat) 3267, 3085, 2859, 1722, 1707, 1468, 1139, 959 | |
| | 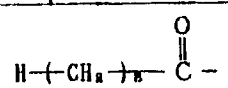 | | | | |

Table 1 (continued)

| Ex. No. | R1 : | R3 : | NMR (solvent used in measurement) ; δ ppm | IR $cm^{-1}$ | Melting Point (°C) |
|---|---|---|---|---|---|
| | R2 : | | | | |
| 31 | H | H | ($CDCl_3$) 0.88 (3H, m), 1.27 (10H, m), 1.65 (4H, m), 2.08 (2H, m), 2.48 (1H, m), 2.73 (1H, m), 4.19-4.65 (6H, m), 6.28 (1H, dd), 7.27 (2H, d), 7.87 (2H, d), 8.09 (1H, s), 9.72 (1H, br) | (KBr) 3037, 2037, 2119, 1774, 1606, 1354, 1278, 953 | 103-104 |
| | 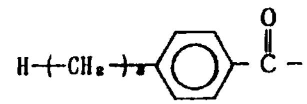 | | | | |
| 32 | H | H | ($CDCl_3$) 0.88 (3H, m), 1.30 (20H, m), 1.63 (2H, m), 2.08 (1H, m), 2.02 (4H, m), 2.33 (2H, m), 2.49 (1H, m), 2.67 (1H, m), 4.16-4.44 (6H, m), 5.35 (2H, m), 6.22 (1H, t), 8.09 (1H, s), 9.03 (1H, b) | (neat) 3269, 3006, 2854, 2120, 1705, 1467, 1411, 1279, | |
| | 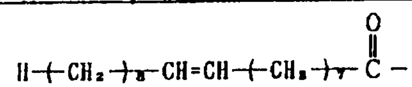 | | | | |
| 33 | H | H | ($CDCl_3$) 0.97 (3H, m), 1.25 (6H, m), 1.57 (6H, m), 2.08 (1H, m), 2.06 (4H, m), 2.32 (2H, m), 2.50 (1H, m), 2.69 (1H, m), 4.20-4.43 (6H, m), 5.36 (4H, m), 6.19 (1H, t), 8.09 (1H, s), 8.20 (1H, br) | (neat) 3085, 2855, 1703, 1477, 1362, 1281, 1132, 761 | |
| | 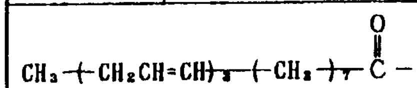 | | | | |
| 34 | H | H | ($CDCl_3$) 1.77 (6H, t), 1.64-1.77 (2H, m), 2.15 (1H, m), 2.51 (1H, m), 2.47 (1H, m), 3.20-3.32 (2H, m), 3.51-3.68 (6H, m), 4.16-4.42 (6H, m), 6.15 (1H, t), 8.03 (1H, s) | (KBr) 3260, 2954, 1696, 1403, 1225, 1176, 915, 818 | 131-133 |
| | 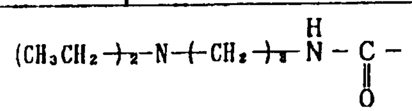 | | | | |
| 35 | H | H | ($CDCl_3$) 2.06 (2H, m), 2.75 (1H, m), 3.51 (2H, s), 3.82-3.34 (8H, m), 4.34-4.18 (6H, m), 6.13 (1H, dd), 6.88 (2H, d), 7.90 (4H, d), 8.03 (1H, s) | (KBr) 3269, 2922, 2852, 2126, 1691, 1423, 1350, 1154 | 166-169 |
| | 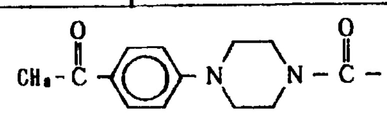 | | | | |

Table 1 (continued)

| Ex. No. | R1 : | R3 : | NMR (solvent used in measurement) ; δ ppm | IR cm-1 | Melting Point (℃) |
|---|---|---|---|---|---|
| | R2 : | | | | |
| 36 | H | H | ($CDCl_3$)2.12(2H,m),2.29(2H,m),2.50(1H,m),2.69(1H,m),3.79-3.25(10H,m),4.45-4.11(8H,m),6.25(1H,t),8.55(1H,s),9.10(1H,bs) | (KBr) 3194,3094,2884,1719,1699,1145,951,784 | 171-174 |
| | 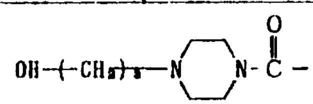 | | | | |
| 37 | H | H | ($CDCl_3$)1.10(1H,m),1.25-1.45(4H,m),1.66(3H,m),1.80(2H,m),1.99(1H,ddd),2.44(1H,t),2.71(1H,ddd),2.76(3H,s),3.85(1H,m),4.19(1H,dd),4.21(1H,dd),4.28-4.38(4H,m),6.09(1H,dd),7.99(1H,bs),8.04(1H,s) | (KBr) 3449,3182,3088,2935,2121,1705,1463,1277 | 131-133 |
| | 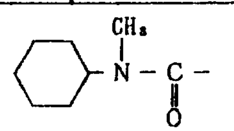 | | | | |
| 38 | H | H | ($CDCl_3$)0.88(3H,t),1.26(10H,s),1.49(2H,m),1.62(2H,br),2.07(1H,dt),2.49(1H,t),2.66(1H,ddd),3.18(2H,m),4.21(1H,dd),4.22(1H,dd),4.32(2H,m),4.42(1H,dd),4.69(1H,m),6.17(1H,t),8.18(1H,s),8.50(1H,bs) | (KBr) 3246,2921,1717,1468,1279,1140,1079 | 92-94 |
| | 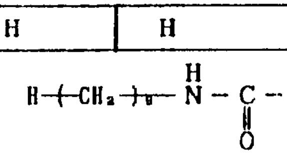 | | | | |
| 39 | H | H | ($CDCl_3$)0.88(3H,t),1.26(16H,s),1.49(2H,m),1.67(2H,br),2.07(1H,dt),2.49(1H,t),2.66(1H,ddd),3.18(2H,m),4.21(1H,dd),4.23(1H,dd),4.31(2H,m),4.42(1H,dd),4.68(1H,m),6.18(1H,t),8.18(1H,s),8.60(1H,bs) | (KBr) 3247,2920,2851,2115,1717,1469,1140,1079 | 101-103 |
| | 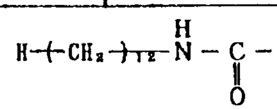 | | | | |
| 40 | H | H | ($CDCl_3$)0.9(3H,t),1.4(2H,sixtet),1.5(2H,quintet),2.1(1H,dt),2.5(1H,t),2.7(1H,ddd),3.2(2H,m),4.21(1H,dd),4.23(1H,dd),4.28-4.33(3H,m),4.42(1H,m),4.7(1H,bs),6.2(1H,t),8.2(1H,s),9.1(1H,bs) | (KBr) 3345,3071,2959,2119,1706,1545,1473 | 90-95 |
| | 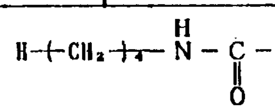 | | | | |

Table 1 (continued)

| Ex. No. | $R^1$ : | $R^2$ : | $R^3$ : | N M R (solvent used in measurement) ; δ ppm | I R $cm^{-1}$ | Melting Point (℃) |
|---|---|---|---|---|---|---|
| 41 | methyl | H | 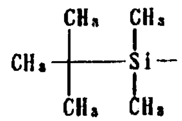 | ($CDCl_3$) 0.094(3H,s), 0.097(3H,s), 0.901(9H,s), 1.86(3H,t), 1.96(1H,ddd), 2.63(1H,ddd), 3.82(1H,dd), 3.94(1H,dd), 4.12(1H,dq), 4.20(1H,dq), 4.24(1H,bd), 4.35(1H,bd), 6.18(1H,dd), 8.26(1H,s), 8.63(1H,bs) | (KBr) 2236, 1703 | 102-104 |
| 42 | methyl | H | H | ($CDCl_3$) 1.86(3H,t), 2.20(1H,dt), 2.52(1H,ddd), 3.78(1H,bd), 3.91(1H,bd), 4.14(2H,t), 4.17(1H,bd), 4.36(1H,dt), 4.41(1H,bs), 6.25(1H,t), 8.70(1H,s) | (KBr) 2249, 1735, 1687 | 151-153 |
| 43 | H | H | 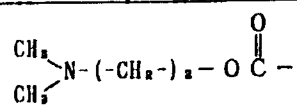 | ($CDCl_3$) 2.24(1H,dt), 2.32(6H,s), 2.52(1H,t), 2.67(2H,m), 4.20(1H,dd), 4.24(1H,dd), 4.33(1H,m), 4.36(1H,m), 4.41(2H,m), 6.17(1H,t), 8.07(1H,s) | (neat) 3303, 3021, 2960, 2118, 1753, 1705, 1463, 1283 | |
| 44 | H | 4-anisoyl | 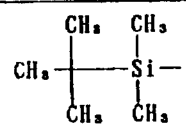 | ($CDCl_3$) 0.116(3H,s), 0.121(3H,s), 0.93(9H,s), 2.02(1H,st), 2.46(1H,t), 2.66(1H,bdd), 3.85(1H,dd), 3.89(3H,s), 3.97(1H,dd), 4.18(1H,dd), 4.21(1H,dd), 4.27 4.37(1H,bd), 6.18(1H,dd), 6.97(2H,d), 7.88(2H,d), 8.34(1H,s) | | |
| 45 | H | 4-anisoyl | H | ($CDCl_3$) 2.29(1H,dt), 2.48(1H,t), 2.68(1H,ddd), 3.88(3H,s), 4.02(1H,dd), 4.18(1H,dd), 4.21(1H,dd), 4.23(1H,bd), 4.43(1H,bdd), 6.25(1H,dd), 6.97(2H,d), 7.89(2H,d), 8.72(1H,bs) | (neat) 3504, 3279, 2366, 1749, 1708, 1676 | |

Table 1 (continued)

| Ex. No. | $R^1$ : | $R^2$ : / $R^3$ : | N M R (solvent used in measurement) ; δ ppm | I R $cm^{-1}$ | Melting Point (℃) |
|---|---|---|---|---|---|
| 46 | H | 2-furoyl / 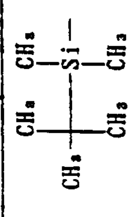 | ($CDCl_3$) 0.12 (3H, s), 0.14 (3H, s), 0.92 (9H, s), 2.05 (1H, dt), 2.47 (1H, t), 2.67 (1H, bdd), 3.85 (1H, dd), 3.96 (1H, dd), 4.18 (1H, dd), 4.22 (1H, dd), 4.28 (1H, bd), 4.37 (1H, bd), 6.18 (1H, t), 6.64 (1H, dd), 7.48 (1H, d), 7.66 (1H, bs), 8.32 (1H, bs) | | |
| 47 | H | 2-furoyl / H | ($CDCl_3$) 2.3 (1H, dt), 2.49 (1H, t), 2.6 (1H, ddd), 3.92 (1H, dd) 4.06 (1H, dd), 4.17 (1H, dd), 4.2-4.3 (1H, m), 4.25 (1H, dd), 4.43-4.47 (1H, m), 6.26 (1H, t), 6.64 (1H, dd), 7.49 (1H, d), 7.66 (1H, bs), 8.7 (1H, bs) | (KBr) 3504, 3288, 2368, 1745, 1717, 1684 | 91-94 |

The anti-cancer activities of the compounds of the present invention and their kinetics in blood were tested.

Pharmacological Test 1 (Anti-cancer Activity)

Each of the compounds of the present invention prepared in Examples 1, 2, 6, 7, 11, 13, 14, 28, 29, 32, 45 and 47 was uniformly suspended in 0.5% aqueous carboxymethylcellulose solution containing 0.1% Tween 80 using an agate mortar. Female $CDF_1$ mice of 6 weeks old were used. As the tumor, Meth A fibrosarcoma was used.

To the right abdomen of each mouse, $2 \times 10^6$ tumor cells were subcutaneously transplanted. On the next day (day 1), the mice were randomly grouped and the suspension was compulsively administered to the mice by using an oral sound for successive 7 days (day 1 - day 7).

On day 14, the tumors were removed and weighed. The tumor growth ratio (%) was calculated according to the following equation:

Tumor Growth Ratio (%) = T/C x 100

wherein T means the average weight of tumor of the treated group and C means the average weight of tumor of the control group. The results are shown in Table 2.

Table 2

| Test Compound (Example No.) | Dose ( mg/kg) | Body Weight Change ( g ) | Tumor Weight Ratio ( T/C : %) |
|---|---|---|---|
| Example 1 | 13.4<br>53.7 | -0.3<br>-4.9 | 67.0<br>33.0 |
| Example 2 | 17.3<br>51.9 | -0.5<br>-4.9 | 16.6<br>7.7 |
| Example 6 | 22.7<br>68.1 | -3.8<br>-4.7 | 28.3<br>2.6 |
| Example 7 | 26.0<br>52.0 | -0.2<br>-4.0 | 38.0<br>25.0 |
| Example 11 | 37.0<br>75.0 | 0.6<br>-2.7 | 58.0<br>31.0 |
| Example 13 | 27.0<br>54.0 | 0.8<br>-2.9 | 57.0<br>37.0 |
| Example 14 | 38.0<br>76.0 | -0.2<br>-4.7 | 32.0<br>15.0 |
| Example 28 | 54.9 | -5.8 | 26.0 |
| Example 29 | 18.0<br>71.9 | -1.2<br>-6.9 | 52.0<br>21.0 |

Table 2 Continued

| Test Compound (Example No.) | Dose (mg/kg) | Body Weight Change (g) | Tumor Weight Ratio (T/C : %) |
|---|---|---|---|
| Example 32 | 36.0<br>72.0 | 0.5<br>−3.8 | 50.0<br>15.0 |
| Example 45 | 28.0<br>84.1 | 1.3<br>−4.5 | 64.6<br>6.9 |
| Example 47 | 25.6<br>76.9 | 0.7<br>−4.4 | 90.3<br>21.7 |
| Control | | 1.0 | 100.0 |

Pharmacological Test 2 (Kinetics in Blood)

Animals

Male SD rats of 6 weeks old were acclimatized for 1 week and 7 weeks old rats were used for the experiments.

Administration of Test Compounds and Extraction

1) Administration of Test Compounds and Collection of Blood

The compounds of the present invention prepared in Examples 1, 2, 7, 9, 13, 27 and 30 were suspended in 0.5% carboxymethylcellulose solution containing 0.1% Tween 80 and the suspension was orally administered to the rats by using an oral sound. The rats were anesthetized with chloroform and 5 ml of blood was collected from abdominal inferior vena cava after opening their abdomina.

2) Extraction

The blood was centrifuged and plasma was collected. To 200 µl of the plasma, 10 µg/200 µl of 5-fluorouracil (used as an internal standard in HPLC) and 4 ml of ethyl acetate were added. The resulting mixture was shaken and then centrifuged. Then 3 ml of the ethyl acetate layer was placed in another test tube and evaporated to dryness under nitrogen gas blow. To the obtained residue, 200 µl of 50% acetonitrile/25 mM sodium phosphate (pH 4.0) was added and the resultant was subjected to ultrasonication. Then the insoluble materials were removed by centrifugation and the supernatant was collected.

Analysis by HPLC

Using a gel permeation column for analysis of nucleic acids (Asahipak GS-320H), 80 µl of the thus

obtained supernatant was subjected to the analysis by HPLC. The flow rate was 0.5 ml/min. and the detection was carried out at 254 nm. As the mobile phase, 50% acetonitrile and 25 mM sodium phosphate (pH 4.0) were used. The quantification of F3Thd was carried out according to absolute calibration method using Chromatocorder 12.

The results are shown in Fig. 1. As shown in Fig. 1, the compounds of the present invention exist in blood in 1 - 4 hours after the administration and even at 8 hours after administration, they existed in a detectable level.

Pharmacological Test 3 (Comparison of Increase in Life Span)

The compound of the present invention prepared in Example 1 was uniformly suspended in 0.5% carboxymethylcellulose solution containing 0.1% Tween 80 using an agate mortar. On the other hand, F3Thd was dissolved in phosphate buffered physiological saline. Female $CDF_1$ mice of 6 weeks old were used. As the tumor, L1210 was used.

To the abdominal cavities of the mice, $5 \times 10^4$ tumor cells were transplanted. On the next day (day 1), the mice were randomly grouped and the suspension was compulsively administered to the mice by using an oral sound for successive 7 days (day 1 - day 7).

The average survival days of each group was determined and the increase in life span (ILS, %) was calculated according to the following equation:

$$ILS (\%) = (T - C)/C \times 100$$

(wherein T means the average survival days of the treated group and C means the average survival days of the control group).

The results are shown in Fig. 2. As can be seen from Fig. 2, the compound of the present invention exhibits higher life span-prolonging effect with lower dose than F3Thd. This was also observed for other compounds of the present invention.

**Claims**

1. A trifluorothymidine derivative of the formula [I]:

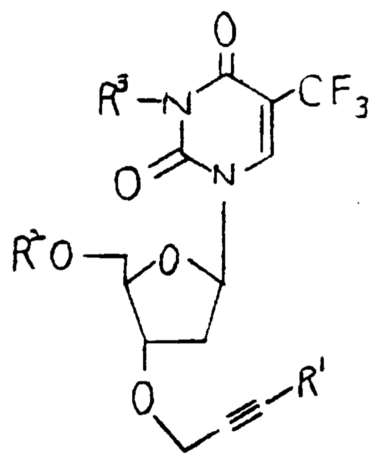

[I]

(wherein $R^1$ represents hydrogen atom or $C_1$ - $C_4$ alkyl group; $R^2$ represents hydrogen atom, $C_1$ - $C_{30}$ saturated or unsaturated alkyl-substituted carbonyl group, $C_1$ - $C_{10}$ alkyl-substituted benzoyl group, $C_1$ - $C_4$ alkyloxycarbonyl group; dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group; $R^3$ represents hydrogen atom, $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group).

2. The trifluorothymidine derivative of claim 1, which is represented by the formula [II]:

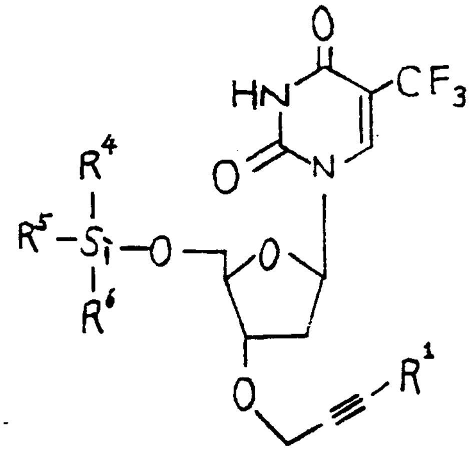

[II]

(wherein $R^1$ is as defined in claim 1 and $R^4$, $R^5$ and $R^6$, the same or different, represent $C_1$ - $C_{20}$ alkyl group or phenyl group).

3. The trifluorothymidine derivative of claim 1, wherein $R^2$ is hydrogen atom, $C_1$ - $C_4$ lower alkyl-substituted carbonyl group, $C_1$ - $C_4$ lower alkoxymethyl group, benzyl group, $C_1$ - $C_4$ lower alkyl-substituted silyl group or trityl group; and $R^3$ is hydrogen atom, benzyl group, benzoyl group, $C_1$ - $C_4$ alkoxy-substituted benzoyl group, furoyl group or $C_1$ - $C_4$ alkyl-substituted carbonyl group.

4. A process for producing a trifluorothymidine derivative of the formula [Ia]:

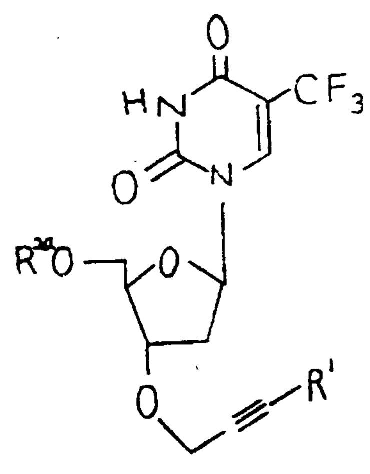

[Ia]

(wherein $R^1$ represents the same meaning as in formula [I], and $R^{2a}$ represents the same meaning as in formula [I] except that it is not hydrogen atom)
comprising reacting a trifluorothymidine derivative of the formula [III]:

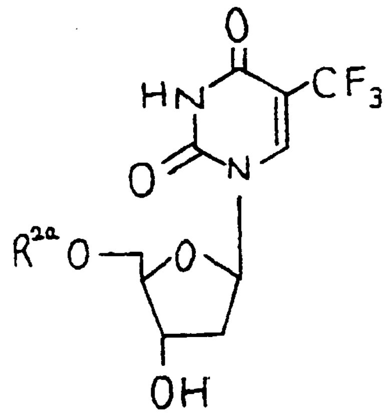

[III]

(wherein $R^{2a}$ represents the same meaning as in formula [Ia]) with a compound of the formula [IV]:

$R^1C \equiv C\text{-}CH_2\text{-}X$ [IV]

(wherein $R^1$ represents the same meaning as in formula [I] and X represents halogen) in the presence of a base.

**5.** A process for producing a trifluorothymidine derivative of the formula [Ib]:

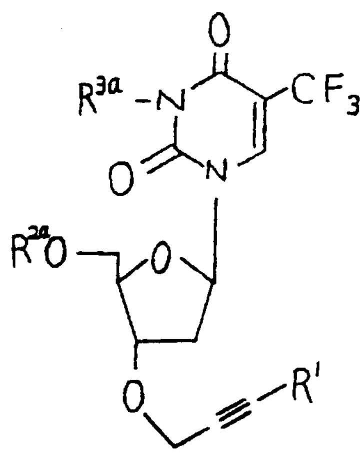

[Ib]

(wherein $R^1$ represents the same meanings as in formula [I], $R^{2a}$ represents the same meaning as in formula [Ia], and $R^{3a}$ represents $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ -$C_4$ alkyl-substituted carbonyl group)
comprising substituting for the hydrogen atom attached to the nitrogen atom at 3-position of the trifluorothymidine derivative of the formula [Ia] set forth in claim 4 a $C_1$ - $C_4$ alkyl group, benzyl group, benzoyl group, $C_1$ - $C_4$ alkyloxy-substituted benzoyl group, furoyl group, $C_1$ - $C_4$ alkyloxymethyl group or $C_1$ - $C_4$ alkyl-substituted carbonyl group.

**6.** A process for producing a trifluorothymidine derivative of the formula [Ic]:

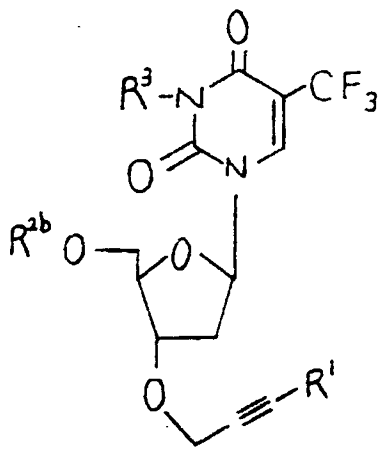

[Ic]

(wherein $R^1$ and $R^3$ represent the same meanings as in formula [I], $R^{2b}$ represents hydrogen atom) comprising reacting a trifluorothymidine derivative of the formula [Id]:

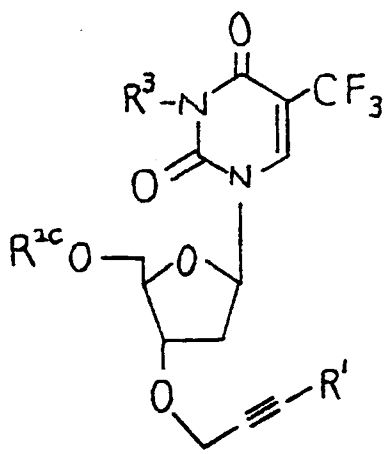

[Id]

(wherein $R^1$ and $R^3$ represent the same meanings as in formula [I], $R^{2c}$ represents $C_1$ - $C_{20}$ alkyl-substituted silyl group, silyl group substituted with $C_1$ - $C_{10}$ alkyl group and/or phenyl group, or trityl group)
with an acid or fluoride ion so as to convert $R^{2c}$ in said trifluorothymidine derivative represented by the formula [Id] to hydrogen atom.

**7.** A process for producing a trifluorothymidine derivative or the formula [Ie]:

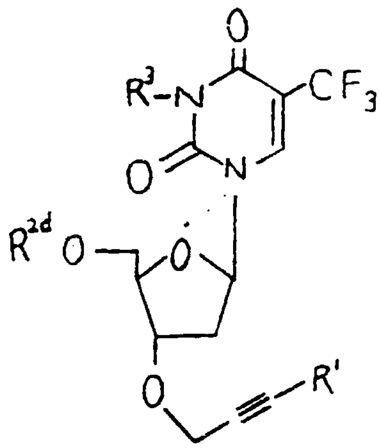

[Ie]

(wherein $R^1$ and $R^3$ represent the same meanings as in formula [I], $R^{2d}$ represents the same meaning as $R^2$ in formula [I] except that it is not hydrogen atom)
comprising substituting for the hydrogen atom represented by $R^{2b}$ of the trifluorothymidine derivative of the formula [Ic] set forth in claim 6 a $C_1$ - $C_{30}$ saturated or unsaturated alkyl-substituted carbonyl group, $C_1$ - $C_{10}$ alkyl-substituted benzoyl group, $C_1$ - $C_4$ alkyloxycarbonyl group, dimethylaminoethyloxycarbonyl group, $C_1$ - $C_4$ alkyloxymethyl group, butoxyethoxyacetyl group, benzyl group, $C_1$ - $C_{20}$ alkyl-substituted silyl group, silyl group substituted with $C_1$ - $C_{10}$ alkyl group and/or phenyl group, $C_1$ - $C_{30}$ cyclic or chain alkyl-substituted carbamoyl group, diethylaminopropylcarbamoyl group, N-alkylpiperazinylacetyl group, N-alkylprolyl group, valyl group, trityl group, alkyl-substituted phosphoryl group or propargyl group.

**8.** An anti-cancer agent comprising effective amount of said trifluorothymidine derivative of claim 1, 2, or 3 and a pharmaceutically acceptable carrier.

**9.** Use of the trifluorothymidine derivative of claim 1, 2 or 3 for the preparation of a pharmaceutical composition for use as an anti-cancer agent.

**10.** Use of the trifluorothymidine derivative of claim 1, 2 or 3 for the preparation of a pharmaceutical composition for use as an anti-tumor agent.

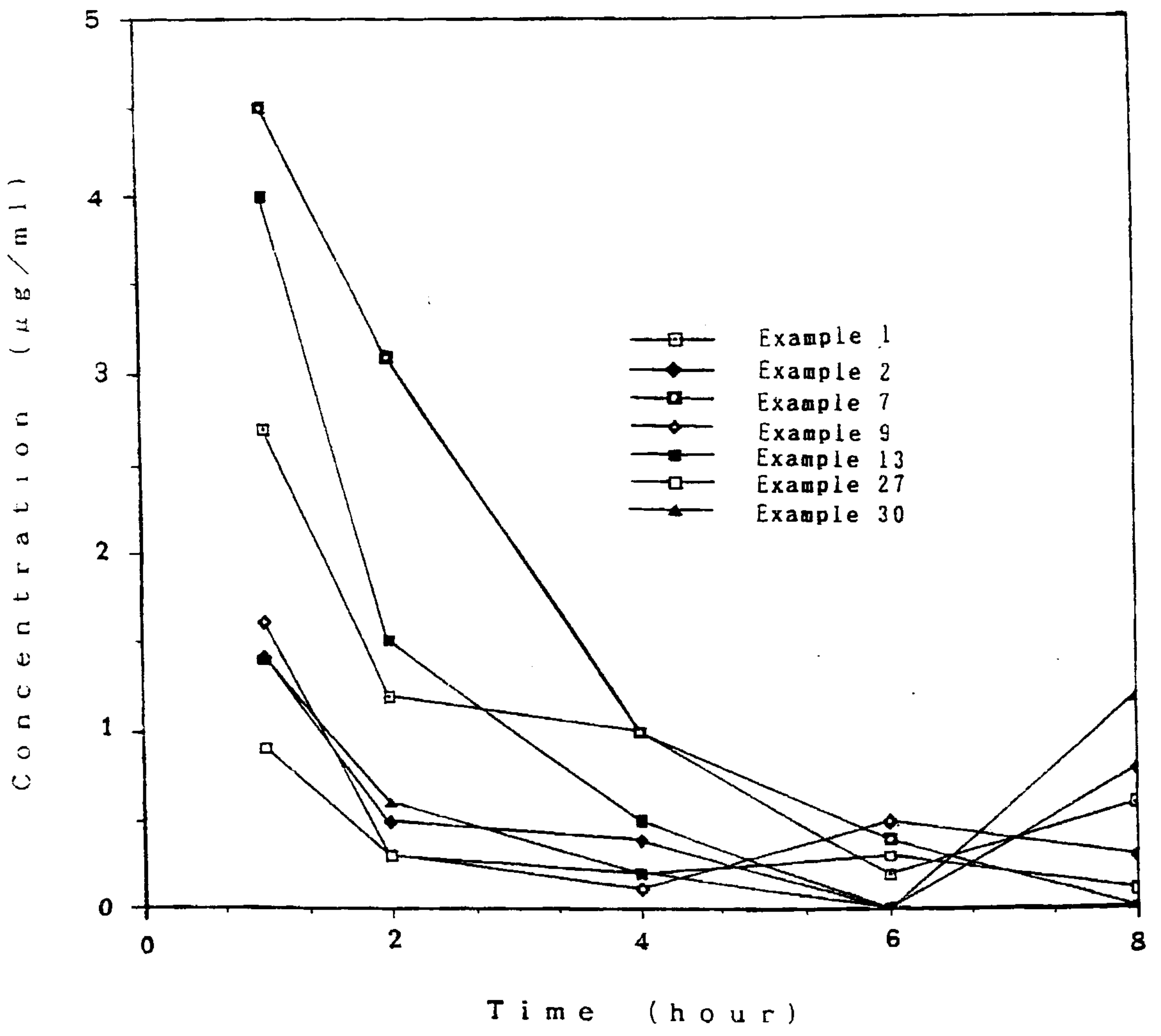
5
4
3
2
1
0
Concentration (μg/ml)
0
2
4
6
8
Time (hour)
Example 1
Example 2
Example 7
Example 9
Example 13
Example 27
Example 30

Fig. 1

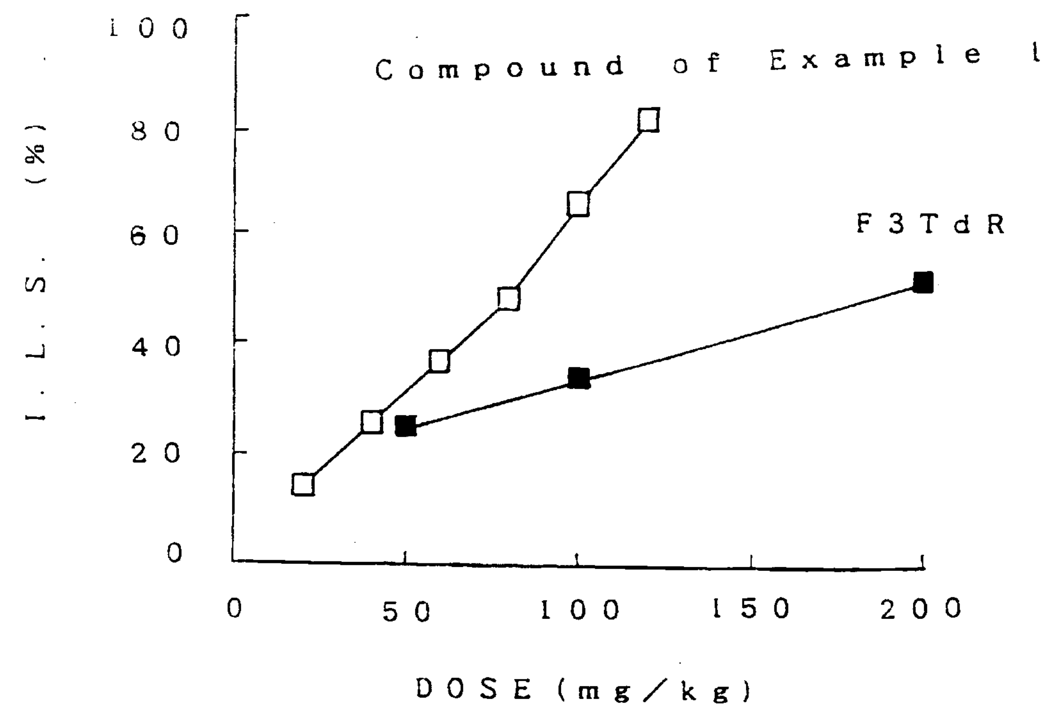
Compound of Example 1
F3TdR
I.L.S. (%)
100
80
60
40
20
0
0
50
100
150
200
DOSE (mg/kg)

Fig. 2

European Patent Office

# EUROPEAN SEARCH REPORT

Application Number

EP 92 10 9574

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| A | CHEMICAL ABSTRACTS, vol. 112, 1990, Columbus, Ohio, US; abstract no. 158845V, K.KOBAYASHI ET AL.: 'Preparation of 5-iodo- or 5 -(trifluoromethyl)-2'-deoxyuridine-5'-phosphates as Antitumour and Antiviral Agents' page 776 ;column 2 ; * abstract * & JP-A-01 261 396 (TAIHO PHARMACEUTICAL CO., LTD....) 18 October 1989 | 1,8-10 | C07H19/06<br>C07H19/10<br>A61K31/70 |
| A | CHEMICAL ABSTRACTS, vol. 110, 1989, Columbus, Ohio, US; abstract no. 24248C, T.UEDA ET AL.: 'Preparation of 5'-O-trityl-2'-deoxy-5-trifluoromethyluridine Derivatives as Intermediates for Antitumour Agents' page 589 ;column 1 ; * abstract * & JP-A-63 179 888 (TAIHO PHARMACEUTICAL CO., LTD....) 23 July 1988 | 1,8-10 | |
| A | EP-A-0 129 984 (TAIHO PHARMACEUTICAL CO., LTD.) * abstract * | 1,8-10 | TECHNICAL FIELDS SEARCHED (Int. Cl.5)<br>C07H<br>A61K |

The present search report has been drawn up for all claims

| Place of search | Date of completion of the search | Examiner |
| --- | --- | --- |
| THE HAGUE | 24 JULY 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document